# EUROPEAN PATENT SPECIFICATION

(11) **EP 3 357 465 B1**
(45) Date of publication and mention of the grant of the patent: **10.06.2020**
(21) Application number: 16878050.0
(22) Date of filing: 01.09.2016
(51) Int. Cl.: A61F 13/494, A61F 13/49, A61F 13/496

(54) **UNDERPANTS-TYPE DISPOSABLE DIAPER**
HOSENÄHNLICHE EINWEGWINDEL
COUCHE JETABLE DE TYPE CULOTTE

(30) Priority: 25.12.2015 JP 2015252975
(43) Date of publication of application: 08.08.2018
(73) Proprietor: Unicharm Corporation, Ehime 799-0111 (JP)
(72) Inventor: SAITO, Kyota, Kanonji-shi Kagawa 769-1602 (JP); NAKNAN, Harutai, Chachoengsao (TH); BOONTHANOM, Jurairat, Chachoengsao (TH)
(74) Representative: Dolleymores
(86) International application number: PCT/JP2016/075687
(87) International publication number: WO 2017/110150

(56) References cited:
- EP-A1- 1 184 017
- EP-A1- 1 495 739
- EP-A1- 2 517 681
- JP-A- 2010 240 110
- JP-A- 2011 250 893
- JP-A- 2015 071 006
- US-A- 5 304 159
- US-A1- 2004 002 690

## Description

### [Technical Field]

The present invention relates to underpants-type disposable diapers.

### [Background Art]

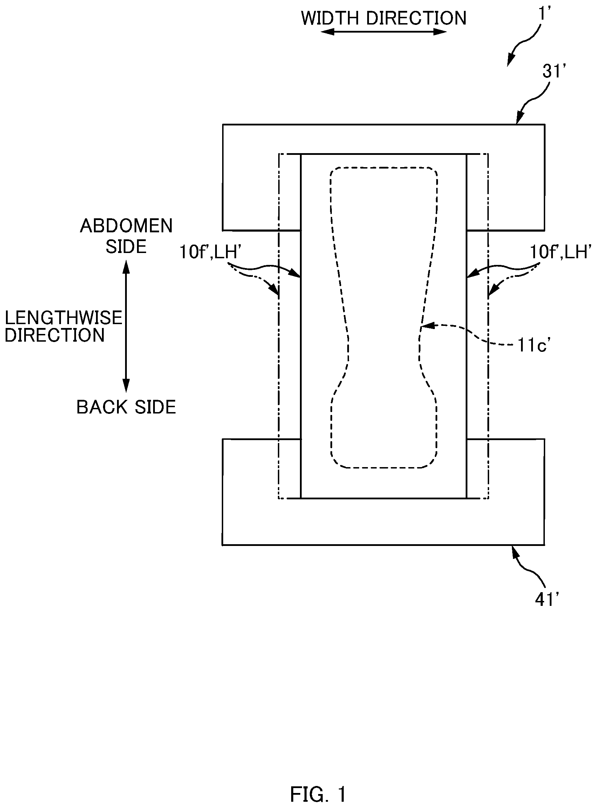

Three-pieced underpants-type disposable diapers have been conventionally used. Such a diaper 1' has a lengthwise direction and a width direction, when in a spread-out state as in Fig. 1, and includes an absorbent main body 10' that absorbs excrement, an abdomen side waist part 31' that overlaps to be joined to an end part of the absorbent main body 10' on one side along the above lengthwise direction, and a back side waist part 41' that overlaps to be joined to an end part of the absorbent main body 10' on the other side along the lengthwise direction. This absorbent main body 10' further includes an absorbent core 11c' that absorbs excrement and a flap part 10f' in a sheet form extending horizontally out from this absorbent core 11c'.

### [Citation List]

### [Patent Literature]

[PTL 1] Japanese Patent Application Laid-open Publication No. 2009-61052

EP 2517681 A1 relates to an underpants-type disposable diaper. First folds extending in the longitudinal direction are defined in portions of gasket cuffs. The first folds are defined along lateral edges of a core of a liquid absorbent structure, and the gasket cuffs being located outboard of the lateral edges are folded along the first folds onto a garment-facing side of an outer sheet. Second folds which extend in the longitudinal direction are formed in containment cuffs. Folded distal ends of the containment cuffs are expanded and folded along the second folds to the garment-facing side of the outer sheet.

EP 1495739 A1 relates to an underpants-type disposable diaper having front and rear waist covering regions and a crotch covering region wherein a bodily discharge absorbing component extends over the crotch covering region into the front and rear waist covering regions. A pair of flaps are provided along the lateral edges of the absorbing component, the flaps being formed by leakage barrier sheets which are folded in the transverse direction of the diaper to present a Z- or inverted Z-shaped cross section. At longitudinally opposite ends of the absorbing component, first and second ends of the flaps folded in a Z- or inverted Z-shape are held in such a folded state by means of an adhesive agent.

### [Summary of Invention]

### [Technical Problem]

The dimension of the above flap part 10f' that configures the leg opening LH' can be extended horizontally outward as indicated with the chain double-dashed lines in Fig. 1 as measures for improving the leak prevention performance at the leg opening LH' of this under-pants type diaper 1'.

However, when the dimension of the flap part 10f' is extended horizontally outward, the leg opening LH' will become small making it difficult for the legs to pass through the leg openings LH' when the wearer wears the diaper 1'.

The present invention has been made in view of the aforementioned conventional issues and a purpose thereof is to let the leg openings achieve excellent leak prevention performance when the underpants-type disposable diaper is worn while allowing the wearer's legs to easily pass through the leg openings when putting on the underpants-type disposable diaper.

### [Solution to Problem]

In order to address the above problem, the present invention provides the underpants-type disposable diaper of independent claim 1. The dependent claims specify preferred but optional features. A primary aspect of the present invention is directed to an underpants-type disposable diaper having a vertical direction and a horizontal direction that intersect each other, including:
an absorbent main body having a lengthwise direction and being folded in two at a predetermined location in the lengthwise direction as well as having the lengthwise direction arranged along the vertical direction;
an abdomen side waist part overlapping and being joined to a non-skin side face of an end part on one side in the lengthwise direction of the absorbent main body; and
a back side waist part overlapping and being joined to a non-skin side face of an end part on another side in the lengthwise direction of the absorbent main body; wherein
the absorbent main body includes an absorbent core that absorbs liquid and a sheet-like flap part that extends horizontally outward with respect to the absorbent core,
the flap part includes on each of the one side and the other side in the lengthwise direction, a Z-shape folded portion that is fixed in a state folded along the horizontal direction in a Z-shape,
   portions located horizontally outward with respect to the absorbent core in the abdomen side waist part and the back side waist part respectively have the Z-shape folded portions of the flap part fixed,
the diaper further comprises a barrier cuff that can stand from a skin side face and is supported by a support that is provided to the skin side face of the flap part,
the flap part includes a middle portion that is not fixed in a folded state at a location between the Z-shape folded portion on the one side and the Z-shape folded portion on the other side, and
a maximum length from the support to a horizontal tip end part of the middle portion, when the middle portion is unfolded and in a horizontally spread state, is greater than a maximum length from the support to a horizontal tip end part of the barrier cuff.

Other features of the present invention will become evident from the description of this specification with reference to the accompanying drawings.

### [Advantageous Effects of Invention]

According to the present invention, the leg openings achieve excellent leak prevention performance when the underpants-type disposable diaper is worn while allowing the wearer's legs to easily pass through the leg openings when putting on the underpants-type disposable diaper.

### [Brief Description of the Drawings]

[Fig. 1] Fig. 1 is an explanatory view showing an issue of an underpants-type disposable diaper 1'.
[Fig. 2] Fig. 2 is a schematic perspective view of the three-piece type diaper 1 seen from the abdomen side, shown as an example of an underpants-type disposable diaper 1 according to a present embodiment.
[Fig. 3A] Fig. 3A is a schematic plan view of the spread-out state diaper 1 seen from the skin side.
[Fig. 3B] Fig. 3B is a sectional view taken along line B-B in Fig. 3A.
[Fig. 3C] Fig. 3C is a sectional view taken along line C-C in Fig. 3A.
[Fig. 3D] Fig. 3D is a sectional view taken along line D-D in Fig. 3A.
[Fig. 3E] Fig. 3E is a sectional view taken along line E-E in Fig. 3A.
[Fig. 4] Fig. 4 is an explanatory diagram showing an area to which the adhesive HMA1 is applied for joining an absorbent main body 10 to an abdomen side waist part 31 and a back side waist part 41.
[Fig. 5] Fig. 5 is a sectional view taken along line V-V in Fig. 4.
[Fig. 6] Fig. 6 is an explanatory view of another example of the area to which the adhesive HMA1 is applied.
[Fig. 7] Fig. 7 is a schematic sectional view illustrating how the middle portion 10fM of the flap part 10f holds back the excrement that had leaked horizontally outward over the barrier cuff LSG.
[Fig. 8A] Fig. 8A is an explanatory view illustrating a step in which the barrier cuff LSG and a Z-shape folded portion 10fZ of the flap part 10f are formed from a back sheet 15.
[Fig. 8B] Fig. 8B is an explanatory view illustrating a step in which the barrier cuff LSG and a Z-shape folded portion 10fZ of the flap part 10f are formed from a back sheet 15.
[Fig. 8C] Fig. 8C is an explanatory view illustrating a step in which the barrier cuff LSG and a Z-shape folded portion 10fZ of the flap part 10f are formed from a back sheet 15.
[Fig. 8D] Fig. 8D is an explanatory view illustrating a step in which the barrier cuff LSG and a Z-shape folded portion 10fZ of the flap part 10f are formed from a back sheet 15.
[Fig. 9] Fig. 9 is an explanatory view illustrating areas to which the adhesives HMA2, HMA3 are applied, the view also being a schematic plan view of the spread-out state diaper 1 seen from the skin side.
[Fig. 10] Fig. 10 is a schematic plan view of the spread-out state diaper 1 for illustrating the type one to type three elastic members 17, 18 and 19.
[Fig. 11] Fig. 11 is a schematic enlarged plan view of the abdomen side waist part 31 of the spread-out state diaper 1 seen from the skin side.
[Fig. 12] Fig. 12 is a schematic plan view of the spread-out state diaper 1 for illustrating the type four elastic member 20.
[Fig. 13] Fig. 13 is a schematic enlarged plan view of the abdomen side waist part 31 of the spread-out state diaper 1 seen from the skin side.
[Fig. 14] Fig. 14 is a schematic enlarged plan view of the back side waist part 41.
[Fig. 15] Fig. 15 is an explanatory view of extended out parts 41ext, 41ext for covering the buttocks.
[Fig. 16A] Fig. 16A is an explanatory view illustrating a modified example of the Z-shape folded portion 10fZ, the view also being a schematic plan view of the spread-out state diaper 1 seen from the skin side.
[Fig. 16B] Fig. 16B is a sectional view taken along line B-B in Fig. 16A.

### [Description of Embodiments]

At least the following matters will become evident from the description of the present specification with reference to the accompanying diagrammatic drawings.

Disclosed is an underpants-type disposable diaper featured to have a vertical direction and a horizontal direction that intersect each other, including:
an absorbent main body having a lengthwise direction and being folded in two at a predetermined location in the lengthwise direction as well as having the lengthwise direction arranged along the vertical direction;
an abdomen side waist part overlapping and being joined to a non-skin side face of an end part on one side in the lengthwise direction of the absorbent main body; and
a back side waist part overlapping and being joined to a non-skin side face of an end part on another side in the lengthwise direction of the absorbent main body; wherein
the absorbent main body includes an absorbent core that absorbs liquid and a sheet-like flap part that extends horizontally outward with respect to the absorbent core,
the flap part includes on each of the one side and the other side in the lengthwise direction, a Z-shape folded portion that is fixed in a state folded along the horizontal direction in a Z-shape, and
portions located horizontally outward with respect to the absorbent core in the abdomen side waist part and the back side waist part respectively have the Z-shape folded portions of the flap part fixed.

According to such an underpants-type disposable diaper, the above described flap parts that configure the leg openings of the diaper respectively include the Z-shape folded portions at locations horizontally outside and on two sides along the lengthwise direction of the absorbent core. Therefore, the leg opening can be suppressed from becoming small due to the tendency of the flap part being folded resulting from the Z-shape folded portion while the flap part secures a substantially large horizontal dimension. And hereby, the gaps between the leg openings and the legs can be effectively filled with the above described flap part that is substantially large in the horizontal direction which as a result allows the leg openings to achieve excellent leak prevention performance when the underpants-type disposable diaper is worn while allowing the wearer's legs to easily pass through the leg openings when putting on the underpants-type disposable diaper.

Further, the Z-shape folded portion of the flap part is located to the skin side with respect to the abdomen side waist part so that this Z-shape folded portion can quickly project to this skin side while the Z-shape folded portion is pressed to the wearer's skin side with the abdomen side waist part. For example, this Z-shape folded portion can quickly project toward and into the concave groin part so to hereby effectively fill the gap between the groin part and the skin. And this effectively contributes to improving the leak prevention performance at the above described leg openings.

It is preferable that in an underpants-type disposable diaper that a predetermined portion including a horizontal outer edge part in the Z-shape folded portion, the predetermined portion including an innermost portion in the lengthwise direction of a portion that overlaps the abdomen side waist part is a non-fixed portion that is not fixed to the abdomen side waist part,
a portion inwardly adjacent to the non-fixed portion in the horizontal direction in the Z-shape folded portion includes a fixed portion that is to be fixed to the abdomen side waist part, and
a portion outwardly adjacent to the non-fixed portion in the lengthwise direction in the Z-shape folded portion includes a fixed portion that is to be fixed to the abdomen side waist part.

According to such an underpants-type disposable diaper, the non-fixed portion of the Z-shape folded portion can separate from the abdomen side waist part. Therefore, the leg openings can be substantially enlarged by an amount created by the non-fixed portion being separated so that the wearer's legs can easily pass through the leg openings as a result.

Further, the portion that is inwardly adjacent in the horizontal direction to the above described non-fixed portion of the Z-shape folded portion and the portion that is outwardly adjacent in the lengthwise direction to the above described non-fixed portion of the Z-shape folded portion respectively include fixing portions that are to be fixed to the abdomen side waist part. Therefore, the Z-shape folded portion can be securely fixed to the abdomen side waist part.

It is preferable that in an underpants-type disposable diaper, the Z-shape folded portion includes an inner fold part that is folded back horizontally inward at a first folding location in the horizontal direction to extend horizontally inward with respect to the first folding location and an outer fold part that is folded back horizontally outward at a second folding location in the inner fold part to extend horizontally outward with respect to the second folding location,
the inner fold part and the outer fold part have faces opposing each other joined, and a horizontally inner portion with respect to the first folding location in the flap part and the inner fold part have faces opposing each other joined as well, in the Z-shape folded portion, and
the outer fold part has a portion extending horizontally outward with respect to the inner fold part.

According to such an underpants-type disposable diaper, the above described Z-shape folded portion that can oppose the wearer's groin part includes an overlapped portion formed by the inner fold part and the outer fold part overlapping each other and this overlapped portion is generally formed thick. Therefore, this overlapped portion can effectively fill the gap created with the skin of the groin part by entering into the concave groin part. And hereby, excellent leak prevention performance can be achieved also at the groin part.

Further, the outer fold part includes a portion that extends horizontally outward with respect to the inner fold part. In other words, the outer fold part includes a portion that extends horizontally outward with respect to the above described overlapped portion; however, this extending portion is a portion where only the outer fold part exists. Therefore, this extending portion can deform flexibly with its low bending rigidity in turn allowing an excellent fitting performance to the wearer's skin. And hereby, this extending portion can function to hold back matters that had excreted from the groin part, which could not be stopped at the above described overlapped portion, and this also effectively contributes to the above described improvement in the leak prevention performance.

It is preferable that in an underpants-type disposable diaper, a predetermined portion that includes a horizontal outer edge part of the extending portion in the outer fold part and that further includes an innermost portion in the lengthwise direction of a portion that overlaps the abdomen side waist part is a non-fixed portion that is not fixed to the abdomen side waist part,
a portion that is outwardly adjacent to the non-fixed portion in the lengthwise direction in the extending portion in the outer fold part includes a fixed portion that is to be fixed to the abdomen side waist part, and
a portion that is inwardly adjacent in the horizontal direction to the non-fixed portion in the Z-shape folded portion includes a fixed portion that is to be fixed to the abdomen side waist part.

According to such an underpants-type disposable diaper, the above described non-fixed portion in the outer fold part can separate from the abdomen side waist part. Therefore, the leg opening can be substantially enlarged by an amount created by the non-fixed portion being separated.

Further, this non-fixed portion can quickly stand from the abdomen side waist part so as a result to effectively function to hold back the excrement from leaking.

Furthermore, the portion that is outwardly adjacent in the lengthwise direction to the above described non-fixed portion in the above described extending portion of the outer fold part and the portion that is inwardly adjacent in the horizontal direction to the above described non-fixed portion in the Z-shape folded portion respectively include fixing portions that are to be fixed to the abdomen side waist part. Therefore, the Z-shape folded portion can be securely fixed to the abdomen side waist part.

It is preferable that in an underpants-type disposable diaper, the absorbent core includes, a constricted part that has a small horizontal dimension with respect to two end parts in the lengthwise direction of the absorbent core, at a location between the two end parts in the lengthwise direction and
the absorbent main body includes a first elastic member along the lengthwise direction to impart to the absorbent main body a contraction force in the lengthwise direction, and
when seen from a skin side the disposable diaper that is in a spread-out state with the absorbent main body opened in the lengthwise direction at the predetermined location to unfold a twofold state of the absorbent main body,
the first elastic member overlaps the absorbent core at the two end parts of the absorbent core and simultaneously the first elastic member is located horizontally outward with respect to the absorbent core without overlapping the absorbent core at the constricted part.

According to such an underpants-type disposable diaper, the absorbent main body in the underpants-type disposable diaper worn by a wearer can be kept from drooping excessively. In other words, the portion where the above described constricted part in the absorbent main body is located and the portions adjacent thereto are most likely to droop downward, however, the first elastic member is located at a position relatively close to the constricted part according to the above described configuration. Therefore, a contraction force can be effectively imparted to the constricted part through the flap part. And hereby, the constricted part can be lifted up to come close to the wearer's skin with this contraction force so that the absorbent main body can be kept from excessively drooping. As a result, a gap can be suppressed from being created between the wearer and the absorbent main body so to improve the leak prevention performance of the underpants-type disposable diaper as a whole.

It is preferable that in an underpants-type disposable diaper, further includes a barrier cuff that can stand from a skin side face is supported by a support that is provided to the skin side face of the flap part, wherein
a second elastic member is provided along the lengthwise direction to a portion that is between the support in the flap part and a horizontally outermost end of the absorbent core to impart a lengthwise contraction force to the flap part.

According to such an underpants-type disposable diaper, the second elastic member is located between the above described support and the horizontally outermost end of the absorbent core and hereby, this second elastic member is located at a position relatively close to the absorbent core. Therefore, contraction force can be effectively imparted to the absorbent core through the flap part. And hereby, when this underpants-type disposable diaper is worn by a wearer, the absorbent core can be lifted up to come close to the wearer's skin with this contraction force even when the absorbent core becomes heavy by absorbing excrement. Thus the absorbent main body can be kept from excessively drooping in an effective manner. As a result, a gap can be suppressed from being created between the wearer and the absorbent main body so to improve the leak prevention performance of the underpants-type disposable diaper as a whole.

It is preferable that in an underpants-type disposable diaper, the second elastic member includes an effective portion that can impart the contraction force and ineffective portions that are each located on two lengthwise sides of the effective portion and that cannot impart the contraction force and
the abdomen side waist part includes a plurality of abdomen side elastic members along the horizontal direction to impart a horizontal contraction force to the abdomen side waist part, and the abdomen side elastic member includes an effective portion that can impart the contraction force and ineffective portions that are each located on two horizontal sides of the effective portion and cannot impart the contraction force,
when seen from a skin side the disposable diaper that is in a spread-out state with the absorbent main body opened in the lengthwise direction at the predetermined location to unfold a twofold state of the absorbent main body,
an intersecting location, where the effective portion of at least one abdomen side elastic member among the plurality of the abdomen side elastic members intersects with the effective portion of the second elastic member, overlaps an area where the abdomen side waist part is joined in the flap part.

According to such an underpants-type disposable diaper, the place of intersection, where the effective portion of the abdomen side elastic member intersects the effective portion of the second elastic member, overlaps the area where the abdomen side waist part is joined in the flap part. Therefore, the contraction force of the abdomen side elastic member can be quickly transmitted to the effective portion of the second elastic member through the abdomen side waist part and the flap part. And hereby, the contraction force of this abdomen side elastic member can also effectively contribute to the contraction force of the second elastic member thereby lifting up the absorbent main body to the skin side through the flap part so to hereby effectively keep the absorbent main body from drooping.

It is preferable that in an underpants-type disposable diaper, a third elastic member to impart the lengthwise contraction force to the flap part is provided in the lengthwise direction and extended across the Z-shape folded portion on the one side and the Z-shape folded portion on the other side,
the third elastic member includes an effective portion that can impart the contraction force and ineffective portions that are each located on two lengthwise sides of the effective portion and that cannot impart the contraction force,
the abdomen side waist part includes a plurality of abdomen side elastic members along the horizontal direction to impart a horizontal contraction force to the abdomen side waist part, and the abdomen side elastic member includes an effective portion that can impart the contraction force and ineffective portions that are each located on two horizontal sides of the effective portion and cannot impart the contraction force,
when seen from a skin side the disposable diaper that is in a spread-out state with the absorbent main body opened in the lengthwise direction at the predetermined location to unfold a twofold state of the absorbent main body,
the effective portion of at least one abdomen side elastic member among the plurality of the abdomen side elastic members does not intersect with the effective portion of the third elastic member at an area where the abdomen side waist part is joined in the flap part.

According to such an underpants-type disposable diaper, when the abdomen side waist part and the back side waist part are pulled horizontally in both sides in order for the wearer to put on the underpants-type disposable diaper, this pulling force is transmitted to the abdomen side elastic member of the abdomen side waist part. And at this time, at least an effective portion of one abdomen side elastic member among the plurality of the abdomen side elastic members and an effective portion of the third elastic member do not intersect each other at the area where the abdomen side waist part is joined in the flap part. Therefore, the transmission of this pulling force is substantially kept from acting between the above described abdomen side elastic member and the above described third elastic member where the respective effective portions do not intersect each other, and hereby the portions proximate the Z-shape folded portion in the flap part is suppressed from spreading horizontally outward even when the above described pulling force is exerted. And hereby, the leg openings are kept from becoming substantially small due to these proximate portions expanding horizontally outward resulting in the leg openings being maintained in a state for the legs of the wearer to pass easily therethrough when the wearer puts on this underpants-type disposable diaper.

It is preferable that in an underpants-type disposable diaper, the back side waist part includes a plurality of back side elastic members along the horizontal direction to impart a horizontal contraction force to the back side waist part, and the back side elastic member includes an effective portion that can impart the contraction force and ineffective portions that are each located on two horizontal sides of the effective portion and cannot impart the contraction force,
when seen from a skin side the disposable diaper that is in a spread-out state with the absorbent main body opened in the lengthwise direction at the predetermined location to unfold a twofold state of the absorbent main body,
at least a part of the effective portion of at least one back side elastic member among the plurality of the back side elastic members is located at an area where the Z-shape folded portion of the flap part is joined in the back side waist part.

According to such an underpants-type disposable diaper, at least a part of the effective portion of the back side elastic member is positioned at an area where the Z-shape folded portion of the flap part is joined at the back side waist part. Therefore, when the abdomen side waist part and the back side waist part of the diaper are pulled horizontally in both sides in order for the wearer to put on the underpants-type disposable diaper, this pulling force is transmitted to the Z-shape folded portion through the above described effective portion of the back side elastic member and hereby the portions proximate the Z-shape folded portion in the flap part is spread horizontally outward. And the buttocks of the wearer can be covered with these expanded portions, as a result.

It is preferable that in an underpants-type disposable diaper, the Z-shape folded portion includes an inner fold part that is folded back horizontally inward at a first folding location in the horizontal direction to extend horizontally inward with respect to the first folding location and an outer fold part that is folded back horizontally outward at a second folding location in the inner fold part to extend horizontally outward with respect to the second folding location,
the inner fold part and the outer fold part have faces opposing each other joined, and a horizontally inner portion with respect to the first folding location in the flap part and the inner fold part have faces opposing each other joined as well, in the Z-shape folded portion,
the flap part includes a liquid impermeable sheet and simultaneously the liquid impermeable sheet is arranged to extend in the horizontal direction toward the inner fold part and the outer fold part while a horizontal tip end part of the outer fold part is a non-positioned portion where the leak impermeable sheet is not arranged, and
the non-positioned portion has a fifth elastic member provided along the lengthwise direction to impart a lengthwise contraction force to the non-positioned portion.

According to such an underpants-type disposable diaper, the leak prevention performance of the inner fold part and the outer fold part are improved with the liquid impermeable sheet of the flap part. Therefore, the leg openings can achieve a more excellent leak prevention performance.

Further, the tip end part of the outer fold part does not have the above described liquid impermeable sheet provided so that the rough texture of the tip end part felt by the skin due to the presence of this liquid impermeable sheet can be averted.

Furthermore, this tip end part can come into intimate contact with the wearer's legs by the contraction force of the above described fifth elastic member and this also effectively contributes to improving the leak prevention performance of the leg openings.

In an underpants-type disposable diaper, a barrier cuff that can stand from a skin side face is supported by a support that is provided to the skin side face of the flap part, wherein
the flap part includes a middle portion that is not fixed in a folded state at a location between the Z-shape folded portion on the one side and the Z-shape folded portion on the other side, and
a maximum length from the support to a horizontal tip end part of the middle portion, when the middle portion is unfolded and in a horizontally spread state, is greater than a maximum length from the support to a horizontal tip end part of the barrier cuff.

According to such an underpants-type disposable diaper, the barrier cuff positioned horizontally inward with respect to the horizontal tip end part at the above described middle portion of the flap part. Further, the maximum length from the above described support to the horizontal tip end part of the barrier cuff is made longer than the maximum length from this support to the horizontal tip end part at the above described middle portion. Therefore, the excrement that had leaked out horizontally outward over the barrier cuff can be held back with the above described tip end part of the above described middle portion. And hereby, the leg openings can achieve a more excellent leak prevention performance.

It is preferable that in an underpants-type disposable diaper, the Z-shape folded portion includes an inner fold part that is folded back horizontally inward at a first folding location in the horizontal direction to extend horizontally inward with respect to the first folding location and an outer fold part that is folded back horizontally outward at a second folding location in the inner fold part to extend horizontally outward with respect to the second folding location,
the flap part includes a middle portion that is not fixed in a folded state at a location between the Z-shape folded portion on the one side and the Z-shape folded portion on the other side, when a portion that is located between the inner fold part on the one side and the inner fold part on the other side in the middle portion is defined as a middle portion inner fold part, and simultaneously a portion that is located between the outer fold part on the one side and the outer fold part on the other side in the middle portion is defined as a middle portion outer fold part,
a single or a plurality of inner fold part elastic members that impart a lengthwise contraction force to the middle portion inner fold part are provided to extend along the lengthwise direction from the one side of the inner fold part to the other side of the inner fold part,
a single or a plurality of outer fold part elastic members that impart a lengthwise contraction force to the middle portion outer fold part are provided to extend along the lengthwise direction from the one side of the outer fold part to the other side of the outer fold part, and
a lengthwise contraction force (N/cm) per unit horizontal length imparted to the middle portion inner fold part by the inner fold part elastic members is greater than the lengthwise contraction force (N/cm) per the unit horizontal length imparted to the middle portion outer fold part by the outer fold part elastic members.

According to such an underpants-type disposable diaper, the middle portion outer fold part is inclined slightly outward since the middle portion inner fold part contracts more along the lengthwise direction compared to the middle portion outer fold part when the above described middle portion of the flap part stands up when the underpants-type disposable diaper is worn. And this together with the Z-fold structure being allowed to spread horizontally outward allows the middle portion outer fold part to come into contact with the wearer's body in a spread-out plane state.

### ===Present Embodiment===

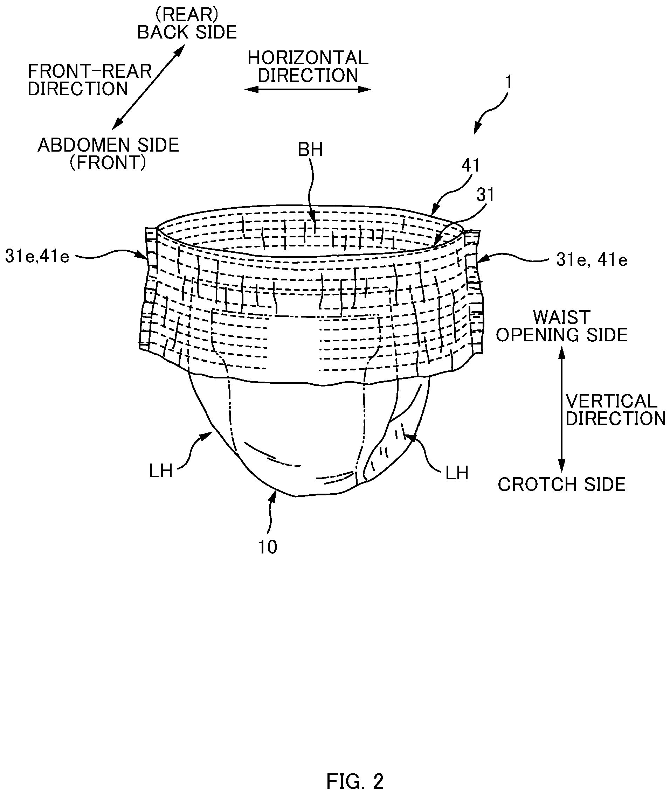

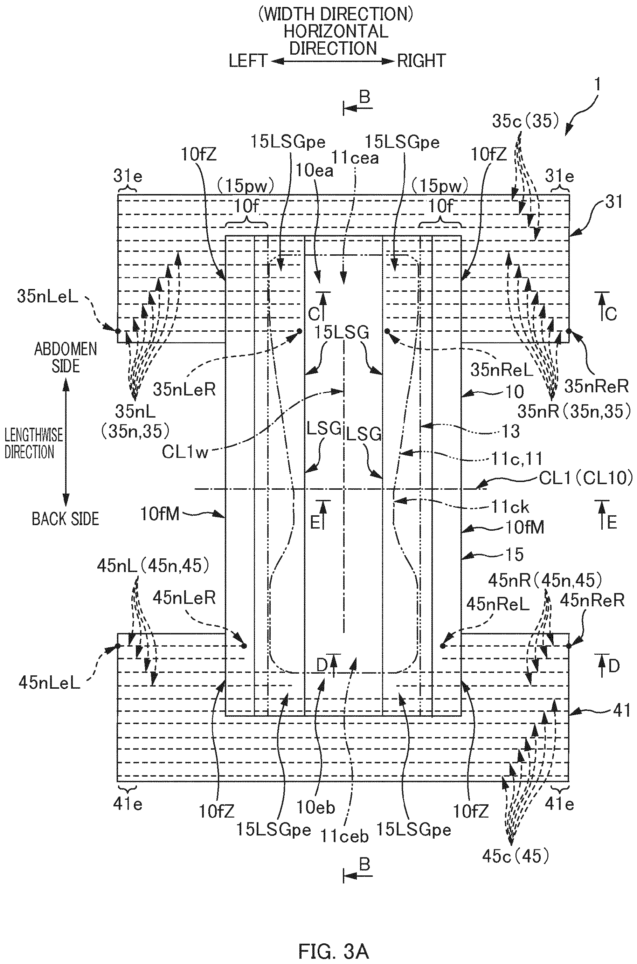

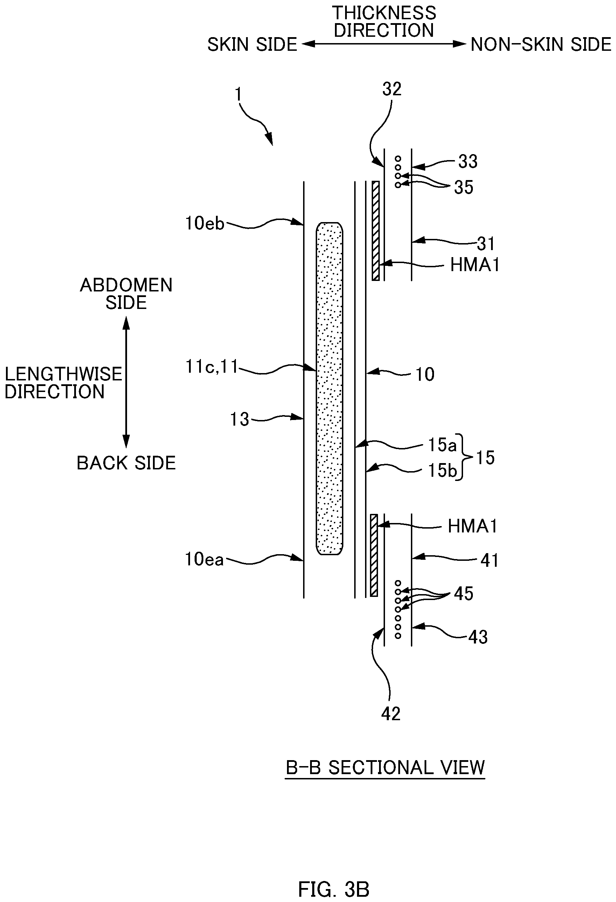

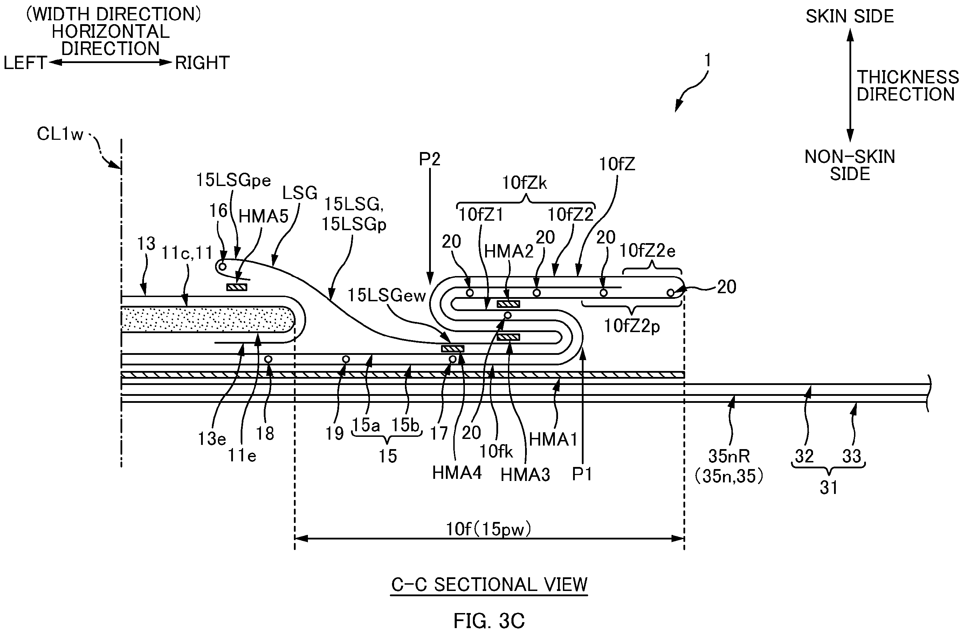

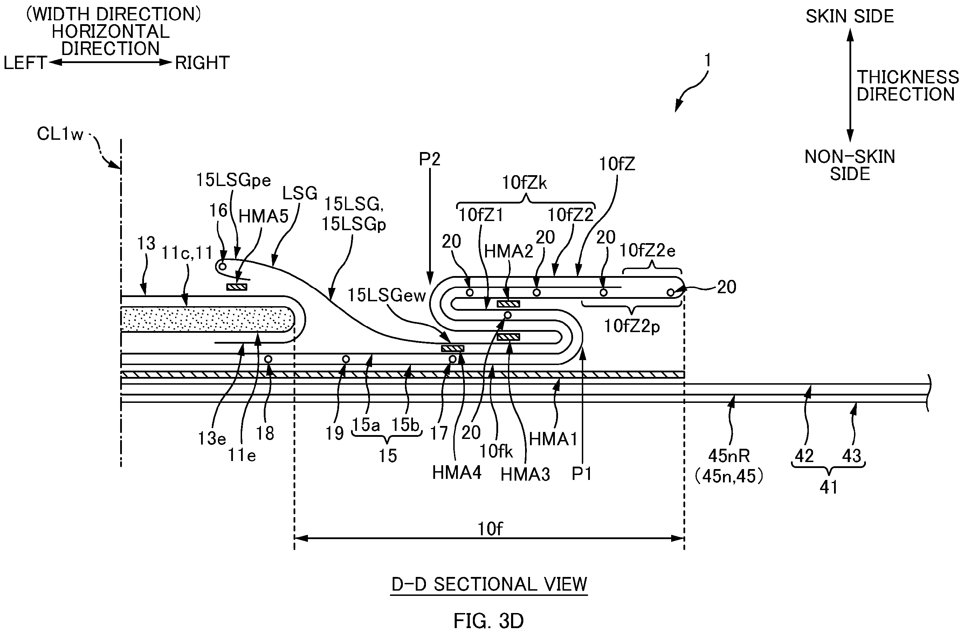

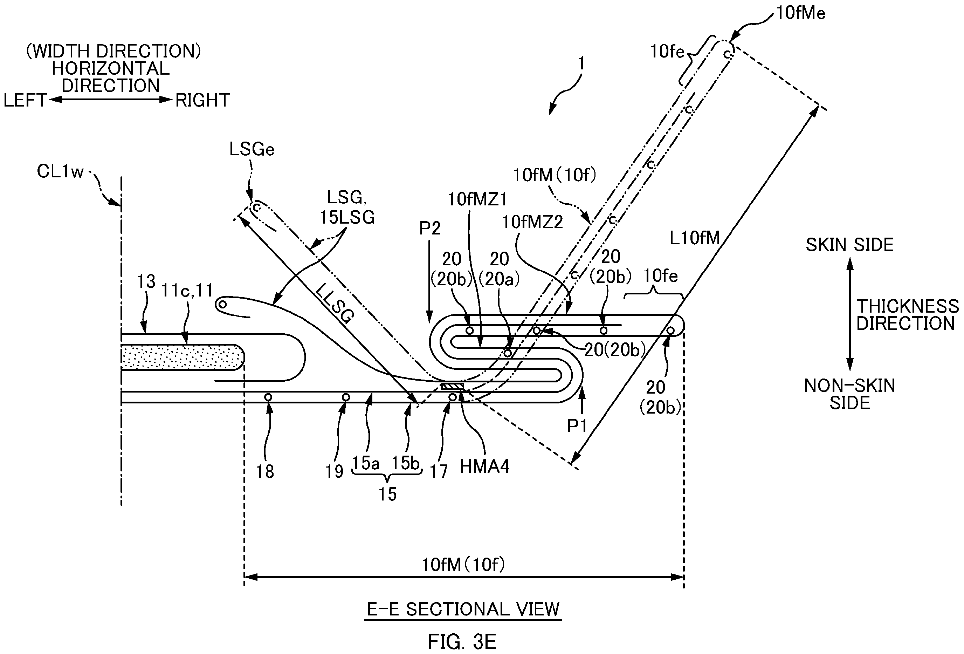

Fig. 2 is a schematic perspective view of the three-piece-type diaper 1 seen from the abdomen side, shown as an example of an underpants-type disposable diaper 1 according to the present embodiment. Further, Fig. 3A is a schematic plan view of the spread-out state diaper 1 seen from the skin side. Furthermore, Fig. 3B is a sectional view taken along line B-B in Fig. 3A, Fig. 3C is a sectional view taken along line C-C in Fig. 3A, Fig. 3D is a sectional view taken along line D-D in Fig. 3A and Fig. 3E is a sectional view taken along line E-E in Fig. 3A.

This diaper 1 in an underpants-type state shown in Fig. 2 includes a vertical direction, a horizontal direction and a front-rear direction as three directions that intersect each other. And in the following description, one side and the other side in the vertical direction in the underpants-type state are also respectively referred to as the "waist opening side" and the "crotch side" and further, the front side and the rear side in the front-rear direction are also respectively referred to as the "abdomen side" and the "back side."

Meanwhile, the diaper 1 in the spread-out state that is shown in Figs. 3A to 3C includes a lengthwise direction, a width direction and a thickness direction as three directions that intersect each other. And in the following description, the one side and the other side in the lengthwise direction in this spread-out state are also respectively referred to as the "abdomen side" and the "back side" and further, the one side and the other side in the thickness direction is also respectively referred to as the "skin side" and the "non-skin side."

Here, the above described width direction in the spread-out state is the direction same as the above described horizontal direction in the underpants-type state. Therefore, the width direction is also referred to as the "horizontal direction" and further, the one side and the other side in the horizontal direction are also referred to as the "left side" and the "right side." Furthermore, the lengthwise direction in the spread-out state comes along the vertical direction in the underpants-type state and the thickness direction in the underpants-type state comes along the front-rear direction in the underpants-type state.

Further, the shapes of the diaper shown in the C-C, the D-D and the E-E sectional views respectively taken along lines C-C, D-D and E-E in Fig. 3A are all line symmetry, that is, dissymmetry with respect to the horizontal center location CLlw of the diaper 1. Therefore, only the right half with respect to the horizontal center location CLlw of the diaper 1 is shown in Figs. 3C, 3D and 3E. And for the portions that are in dissymmetry of the various portions appropriately illustrated in the description will have only the right portion that represent the right and the left side portion described and the left portion will be omitted.

This diaper 1 is a so-called three-pieced type diaper so that in the spread-out state in Fig. 3A, this diaper 1 includes as the first component an absorbent main body 10 that absorbs excrement, as the second component an abdomen side waist part 31 that is in a horizontally long band shape, and as the third component a back side waist part 41 that is in a horizontally long band shape. Specifically, when the abdomen side waist part 31 and the back side waist part 41 are placed parallel with a distance therebetween in the lengthwise direction, the absorbent main body 10 is laid across between the two parts 31, 41 with the lengthwise end parts 10ea, 10eb of the absorbent main body 10 joined and fixed to the respective waist parts 31, 41 closest thereto so to have an outer appearance in a substantially H-shape planar form.

And from this substantially H-shape planar form in a spread-out state, an underpants-type disposable diaper formed with a waist opening BH and a pair of leg openings LH, LH as shown in Fig. 2 are made by folding the absorbent main body 10 in two at a predetermined location CL10, which is regarded as the folding position, in the lengthwise direction of the absorbent main body 10, which is the location that corresponds to the center location CL1 in the lengthwise direction of the diaper 1. And the waist parts 31, 41 are connected to be in an annular form when the waist parts 31, 41 that oppose each other in the two-folded state are joined by welding and the like at the horizontal end parts 31e, 41e.

As is clear from the above description, a "spread-out state" is a state where the joining of the waist parts 31, 41 are released at the horizontal end parts 31e, 41e of the diaper 1 in the underpants-type state shown in Fig. 2, and having the two-folded absorbent main body 10 opened (i.e., the state shown in Fig. 3A) along the lengthwise direction at the above described predetermined location CL10 (CL1.)

Further in the following, for both the spread-out state and the underpants-type state, the spread-out state of the diaper 1 assuming that the contraction force imparted by the later described elastic members 16, 17, 18, 19, 20, 35 and 45 for imparting elasticity to the diaper 1 does not exist at all is called the "pattern state". And the state where no external force is imparted to the diaper 1, that is, the state where the above diaper 1 is contracted with the contraction force by the above described elastic members 16, 17, 18, 19, 20, 35 and 45 is called the "natural state . " By the way, Figs. 3A to 3D show the spread-out state diaper 1 in the pattern state.

In the spread-out state shown in Figs. 3A and 3B, the absorbent main body 10 is in a substantially rectangular shape in a plan view. And the lengthwise direction of the absorbent main body 10 is arranged along the lengthwise direction of the spread-out state diaper 1. Here, the lengthwise direction of the diaper 1 and the lengthwise direction of the absorbent main body 10 being directed in the same direction will both be called the "lengthwise direction" with no distinction between the two.

The absorbent main body 10 includes an absorbent body 11, a top sheet 13 provided to cover the absorbent body 11 from the skin side, and a back sheet 15 provided to cover the absorbent body 11 from the non-skin side.

The absorbent body 11 includes a liquid absorbent core 11c and an unillustrated core wrapping sheet that covers the outer circumferential face of the absorbent core 11c. The absorbent core 11c is a formed body created in a substantially hourglass form in a plan view as an example of a predetermined shape of a certain liquid absorbent material. In other words, the absorbent core 11c includes at a location between the lengthwise end parts 11cea, 11ceb a constricted part 11ck having a horizontal dimension smaller than that of the two lengthwise end parts 11cea, 11ceb of the absorbent core 11c. Liquid absorbent fiber such as pulp fiber or liquid absorbent particles such as high-absorbent polymer (so-called SAP) and the like can be exemplified as the liquid absorbent material. Further, a liquid permeable sheet such as tissue paper or nonwoven fabric and the like can be used for the core wrapping sheet, however, this core wrapping sheet may be omitted. Further, the shape of the absorbent core 11c is not at all limited to the above described substantially hourglass shape in plan view and other shapes may do.

The top sheet 13 is a liquid permeable sheet made of nonwoven fabric and the like that has a planar size which extends out from the absorbent body 11 in the lengthwise direction and the horizontal direction. And as shown in Figs. 3B and 3C, this sheet 13 covers the skin side face the absorbent body 11 while the horizontal end parts 13e of this sheet 13 is curled horizontally inward at the non-skin side locations of the absorbent body 11 so to thereby cover the horizontal ends parts 11e at the non-skin side face of the absorbent body 11 and fixed with unillustrated adhesives and the like.

Meanwhile, the back sheet 15 is also a sheet that has a planar size which extends out from the absorbent body 11 in the lengthwise direction and the horizontal direction as shown in Figs. 3A to 3C. And here, a two-layered structure sheet 15 including a liquid impermeable leak prevention sheet 15a made of polyethylene film, polypropylene or the like and an outer covering sheet 15b made of nonwoven fabric that is joined to the non-skin side of the leak prevention sheet 15a is used (see Figs. 3B and 3C.) And as shown in Fig. 3C, the horizontal center part on the skin side face of the back sheet 15 has an absorbent body 11 covered with the top sheet 13 placed thereon and joined with unillustrated adhesives and the like so that the absorbent main body 10 is substantially formed.

And as illustrated in Figs. 3A and 3C, this back sheet 15 is extensively extended horizontally outward to both sides with respect to the absorbent core 11c and hereby, the portions extending out 15pw respectively form the sheet form flap parts 10f that configure the leg openings LH, which will be described later. Further, this back sheet 15 forms the barrier cuffs LSG for avoiding leaks from the sides, which also will be described later.

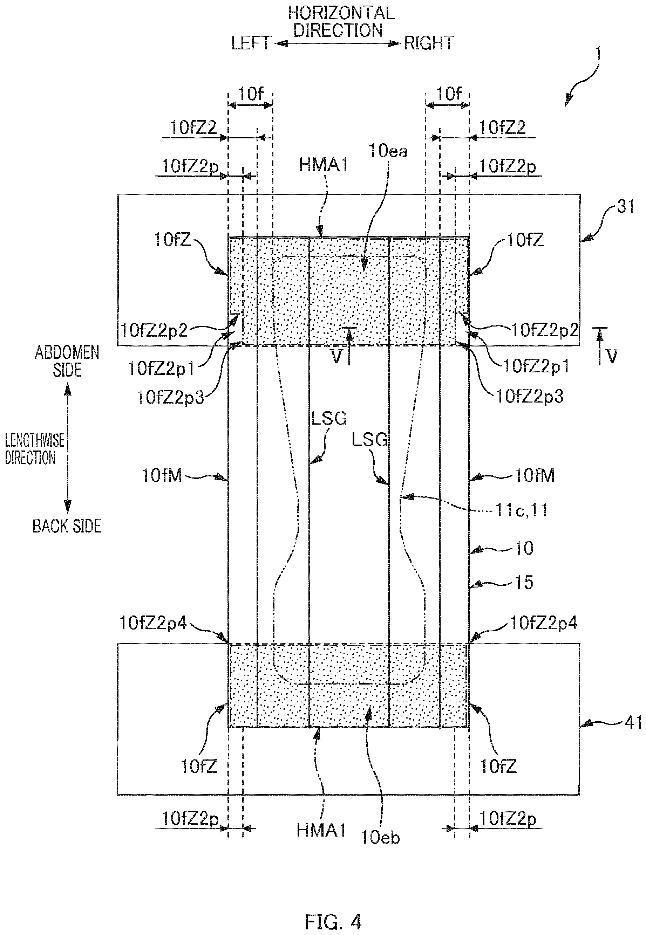

As shown in Fig. 3A, the abdomen side waist part 31 is a horizontally long sheet member in a substantially rectangular shape in plan view and is made with a soft sheet material such as nonwoven fabric and the like. In this example, as shown in Figs. 3B and 3C, the abdomen side waist part 31 is formed by joining the nonwoven fabrics 32, 33 laid over the other. And as shown in Fig. 3A, the horizontal center part of the abdomen side waist part 31 overlaps the non-skin side face of the abdomen side lengthwise end part 10ea of the absorbent main body 10 and joined with adhesives HMA1 (see the dotted area in Fig. 4.)

Further, as shown in Figs. 3A to 3C, the two pieces of nonwoven fabrics 32, 33 in relation with the abdomen side waist part 31 has therebetween a plurality of horizontal elastic members 35, 35 ... such as elastic strings that are arranged and inserted side by side along the lengthwise direction of the diaper 1 while in a horizontally stretched state to be joined and fixed to the nonwoven fabrics 32, 33. And hereby, a horizontal contraction force is imparted to the abdomen side waist part 31. That is, the abdomen side waist part 31 is imparted elasticity in the horizontal direction. Further in this example, as shown in Fig. 3A, a part (e.g., center part in the horizontal direction) of the portion of the abdomen side waist part 31 that overlaps the absorbent body 11 has the elastic members 35 in a discontinuous state for the purpose of keeping wrinkles from being created to the absorbent body 11 and hereby, elasticity is not imparted to this portion. In other words, the elastic members 35, 35 ... that are positioned on the waist opening BH side in the abdomen side waist part 31 are assigned as the continuous elastic members 35c, 35c ... that are continuous along the entire horizontal length whereas the elastic members 35, 35 ... that are positioned on the crotch side in the same abdomen side waist part 31 are assigned as the discontinuous elastic members 35n, 35n ... that are discontinuous at the center part in the horizontal direction. More specifically, the latter discontinuous elastic members 35n has left side elastic members 35nL that are located on the left side with respect to the discontinuous location at the horizontal center and right side elastic members 35nR that are located on the right side with respect to the above discontinuous location. And the left end part 35nLeL of the left side elastic member 35nL is located at the left side end part 31e of the abdomen side waist part 31 while the right end part 35nLeR of the same is located at the left end part of the absorbent core 11c. Additionally, the right end part 35nReR of the right side elastic member 35nR is located at the right side end part 31e of the abdomen side waist part 31 while the left end part 35nReL of the same is located at the right end part of the absorbent core 11c. However, there is no limitation to this.

As shown in Fig. 3A, the back side waist part 41 is a horizontally long sheet member in a substantially rectangular shape in plan view made with a soft sheet material such as nonwoven fabric and the like. In this example, as shown in Figs. 3B and 3C, the back side waist part 41 is formed by joining the nonwoven fabrics 42, 43 laid over the other. And as shown in Fig. 3A, the horizontal center part of the back side waist part 41 overlaps the non-skin side face of the back side lengthwise end part 10eb of the absorbent main body 10 to be joined with adhesives HMA1 (see the dotted area in Fig. 4.)

Further, as shown in Figs. 3A to 3C, the two pieces of nonwoven fabrics 42, 43 in relation with the back side waist part 41 has therebetween a plurality of horizontal elastic members 45, 45 ... such as elastic strings that are arranged and inserted side by side along the lengthwise direction of the diaper 1 while in a horizontally stretched state to be joined and fixed to the nonwoven fabrics 42, 43. And hereby, a horizontal contraction force is imparted to the back side waist part 41. That is, the back side waist part 41 is imparted elasticity in the horizontal direction. Further in this example, as shown in Fig. 3A, a part of the back side waist part 41 that overlaps the absorbent body 11 (e.g., center part in the horizontal direction) has the elastic members 45, 45 ... in a discontinuous state for the purpose of keeping wrinkles from being created to the absorbent body 11 and hereby, elasticity is not imparted to this portion. In other words, the elastic members 45, 45 ... that are positioned on the waist opening BH side in the back side waist part 41 are assigned as the continuous elastic members 45c, 45c ... that are continuous along the entire horizontal length whereas the elastic members 45n, 45n ... that are positioned on the crotch side in the same back side waist part 41 are assigned as the discontinuous elastic members 45n, 45n ... that are discontinuous at the center part in the horizontal direction. More specifically, the latter discontinuous elastic members 45n has left side elastic members 45nL that are located on the left side with respect to the discontinuous location at the center along the horizontal direction and right side elastic members 45nR that are located on the right side with respect to the above discontinuous location. And the left end part 45nLeL of the left side elastic member 45nL is located at the left side end part 41e of the back side waist part while the right end part 45nLeR of the same is located at the left end part of the absorbent main body 10. Additionally, the right end part 45nReR of the right side elastic member 45nR is located at the right side end part 41e of the back side waist part 41 while the left end part 45nReL of the same is located at the right end part of the absorbent main body 10. However, there is no limitation to this.

Hereinabove, description of the main components 10, 31, 41 of the diaper 1 was given, however, the present embodiment is devised such that the leg openings LH achieve excellent leak prevention performance when the underpants-type disposable diaper 1 is worn while the wearer' s legs are allowed to easily pass through the leg openings LH when putting on the underpants-type disposable diaper 1 shown in Fig. 2. The following gives the descriptions of this devise.

The absorbent main body 10 includes flap parts 10f, 10f on the respective horizontal sides, as shown in Fig. 3A. The flap part 10f is for configuring the leg opening LH. And this flap part 10f is made with a soft deformable sheet since the flap part 10f is formed with a portion of the back sheet 15 that extends horizontally outward with respect to the absorbent core 11c.

Further as shown in Figs. 3A, 3C and 3D, the flap part 10f includes along the lengthwise direction on each of the abdomen side and the back side the Z-shape folded portions 10fZ, 10fZ that are fixed in states folded horizontally to be in a Z-shape. And an abdomen side Z-shape folded portion 10fZ and a back side Z-shape folded portion 10fZ are respectively fixed with adhesives HMA1 to the abdomen side waist part 31 and the back side waist part 41 at portions located horizontally outward with respect to the absorbent core 11c.

In this way, the leg openings LH can be suppressed from becoming excessively small based on the tendency of the flap parts 10f being folded by the abdomen and back side Z-shape folded portions 10fZ, 10fZ along the lengthwise direction while the flap parts 10f secure substantially large horizontal dimensions when the flap parts 10f are spread in the horizontal direction.

In other words, in the flap part 10f of Fig. 3A, the portion 10fM (hereinafter also called middle portion 10fM) that is located between the abdomen side Z-shape folded portion 10fZ and the back side Z-shape folded portion 10fZ is a portion 10fM that is not fixed in a Z-shape folded state in the flap part 10f. And this middle portion 10fM is imparted some tendency to be folded by the above described Z-shape folded portions 10fZ, 10fZ that are located on both sides thereof. And hereby, the leg openings LH are suppressed from becoming excessively small. Therefore, this diaper 1 allows the wearer's legs to easily pass through the leg openings LH when the wearer wears this diaper 1. Meanwhile, this middle portion 10fM is allowed to be horizontally spread as indicated with the chain double-dashed lines in Fig. 3E by being released from a folded state so to hereby allow the horizontal dimension of this middle portion 10fM to be increased. Therefore, the gaps between the leg openings LH and the legs are effectively filled with these large flap parts 10f when the diaper 1 is worn. And as a result, these leg openings LH can achieve an excellent leak prevention performance.

Further as described above, the dimension of the flap part 10f being substantially increased allows the standing height of the flap part 10f from the absorbent core 11c to be efficiently secured (Fig. 3E) as well as the area of the flap part 10f at the location of the wearer's buttocks to be secured so that the buttocks can be certainly covered. And by securing the standing height and the area at the buttocks, the space, which is formed to a region, surrounded by the right and left flap parts 10f, 10f of the absorbent main body 10, for containing excrement is increased so that this space can be widely secured. And this also effectively contributes to the above described improvement of leak prevention performance.

In this example, the Z-shape folded portions 10fZ are formed in the following manner. Firstly, as shown in Figs. 3C and 3D, the portion that extends horizontally out from the absorbent core 11c in the back sheet 15 is folded back horizontally inward as well as to the skin side at the first folding location P1 in the horizontal direction. And hereby, an inner fold part 10fZ1 that extends horizontally inward with respect to this first folding location P1 is formed. Further, this inner fold part 10fZ1 is folded back horizontally outward as well as to the skin side at the second folding location P2 that is located horizontally outward with respect to the absorbent core 11c. And hereby, an outer fold part 10fZ2 that extends horizontally outward with respect to the second folding location P2 is formed. And the inner fold part 10fZ1 and the outer fold part 10fZ2 are joined at the faces opposing each other with adhesives HMA2, and also the inner fold part 10fZ1 and the horizontally inner portion 10fk of the flap part 10f with respect to the first folding location P1 are joined at the faces opposing each other with adhesives HMA3. And hereby, the inner fold part 10fZ1 and the outer fold part 10fZ2 are fixed in a Z-shape folded state.

And according to such Z-shape folded portions 10fZ, excrement leakage from the wearer's groin part can be effectively averted. In other words, as shown in Fig. 3C, at first the abdomen side Z-shape folded portion 10fZ is located on the skin side with respect to the abdomen side waist part 31. Therefore, this Z-shape folded portion 10fZ can quickly project toward this skin side while being pushed toward the wearer's skin by the abdomen side waist part 31. Further, this Z-shape folded portion 10fZ includes an overlapping portion 10fZk that is formed with the inner fold part 10fZ1 and the outer fold part 10fZ2 being overlapped and this overlapping portion 10fZk is generally made thick. This Z-shape folded portion 10fZ is a portion that can oppose the groin part of the wearer (Fig. 3A.) Therefore, the overlapping portion 10fZk entering the concave groin part allows the gap formed between the groin part and the skin to be effectively filled so to hereby achieve an excellent leak prevention performance at also the groin part.

As can be seen from referring to Fig. 3C, the outer fold part 10fZ2 in this example includes a portion 10fZ2p that extends horizontally outward with respect to the inner fold part 10fZ1. In other words, the outer fold part 10fZ2 includes a portion 10fZ2p that extends horizontally outward with respect to the above described overlapping portion 10fZk, however, this portion 10fZ2p that extends outward is a portion of the outer fold part 10fZ2 that exists alone. Therefore, this portion 10fZ2p extending outward can deform flexibly based in its low bending rigidity so that an excellent fitting performance to the wearer's skin can be achieved. And hereby, this outward extending portion 10fZ2p can function to hold back the excrement that could not be stopped at the above described overlapping portion 10fZk and had leaked from the groin part, and this also effectively contributes to improving the above described leak prevention performance.

Further, providing this Z-shape folded portion 10fZ to the abdomen side in the lengthwise direction can narrow the width (horizontal dimension) of the flap part 10f at a location of the wearer's groin where the width (horizontal dimension) is narrowed toward the crotch so that the flap part 10f can be made to appropriately abut against this groin part. And this also effectively contributes to improving the above described leak prevention performance.

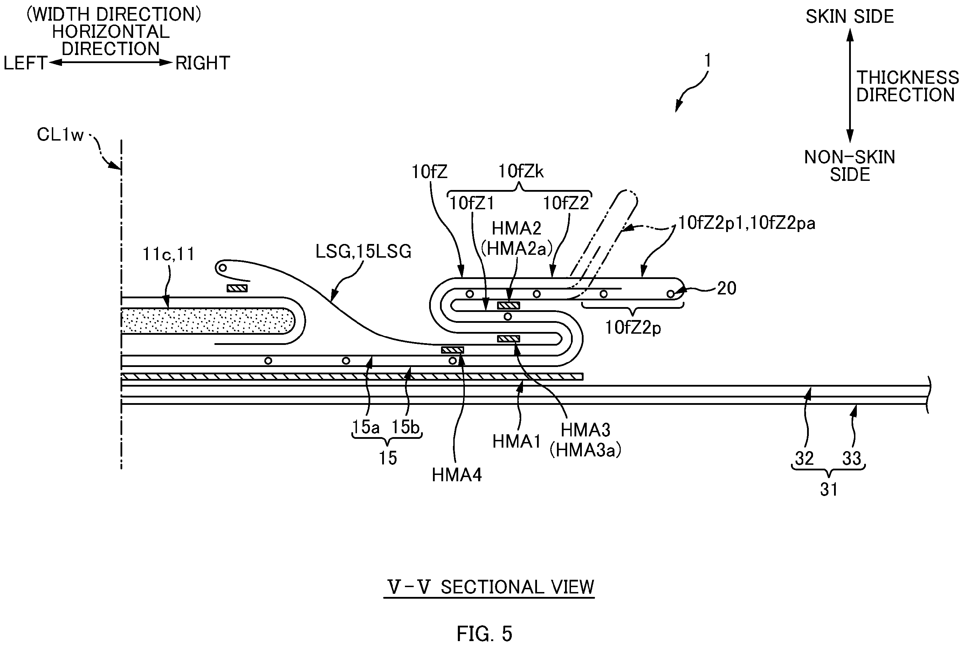

Fig. 4 is an explanatory diagram showing an area to which the adhesive HMA1 is applied for joining an absorbent main body 10 to an abdomen side waist part 31 and a back side waist part 41. In this Fig. 4, the adhesive HMA 1 application area is surrounded by chain double-dashed lines and has the area dotted. Further, Fig. 5 is a sectional view taken along line V-V in Fig. 4.

As illustrated in Fig. 4, when the abdomen side end part 10ea of the absorbent main body 10 is joined with adhesive HMA1 to the skin side face of the abdomen side waist part 31, a large portion of the abdomen side Z-shape folded portion 10fZ is fixed with adhesive HMA1 to the skin side face of the abdomen side waist part 31. However in this example, a part of the Z-shape folded portion 10fZ is not fixed. In other words, a predetermined portion 10fZ2p1 including the horizontal outer edge part in the above described portion 10fZ2p that extends out in the outer fold part 10fZ2, that is, the above described predetermined portion 10fZ2p1 including the innermost side portion in the lengthwise direction of the portion that overlaps the abdomen side waist part 31 is the portion to which adhesive HMA1 is not applied, that is, this part is made a non-fixed portion 10fZ2p1 that is not fixed to the abdomen side waist part 31 with adhesive HMA1.

Therefore, as shown in Fig. 5, this non-fixed portion 10fZ2p1 can be separated from the abdomen side waist part 31 and so the leg openings LH can be made substantially larger by an amount of this portion. And this also effectively contributes to allowing the legs to easily pass through the leg openings LH. Further this non-fixed portion 10fZ2p1 can quickly stand from the abdomen side waist part 31 as shown in the chain double-dashed lines in Fig. 5. Thus this can effectively function to hold back excrement from leaking.

Further, as shown in Fig. 4, in the above described portion 10fZ2p that extends out, the portion 10fZ2p2 that is outwardly adjacent in the lengthwise direction to the non-fixed portion 10fZ2p1 and in the Z-shape folded portion 10fZ, the portion 10fZ2p3 that is inwardly adjacent in the horizontal direction to the non-fixed portion 10fZ2p1 respectively include fixed portions that are to be fixed to the abdomen side waist part 31 with adhesive HMA1. Therefore, the Z-shape folded portion 10fZ can be securely fixed to the abdomen side waist part 31.

Meanwhile, as shown in Fig. 4, the back side Z-shape folded portion 10fZ that is located on the back side waist part 41 does not have a non-fixed portion 10fZ2p1 such as that described above. In other words, a predetermined portion 10fZ2p4 including the horizontal edge part in the above described portion 10fZ2p that extends outward in the outer fold part 10fZ2, that is, the above described portion 10fZ2p4 including the innermost side portion in the lengthwise direction in the portion that overlaps the back side waist part 41 includes a fixed portion that is fixed to the back side waist part 41 with adhesive HMA1. And hereby, the flap part 10f can be easily maintained in a horizontally spread state so that this flap part 10f sufficiently covers the wearer's buttocks.

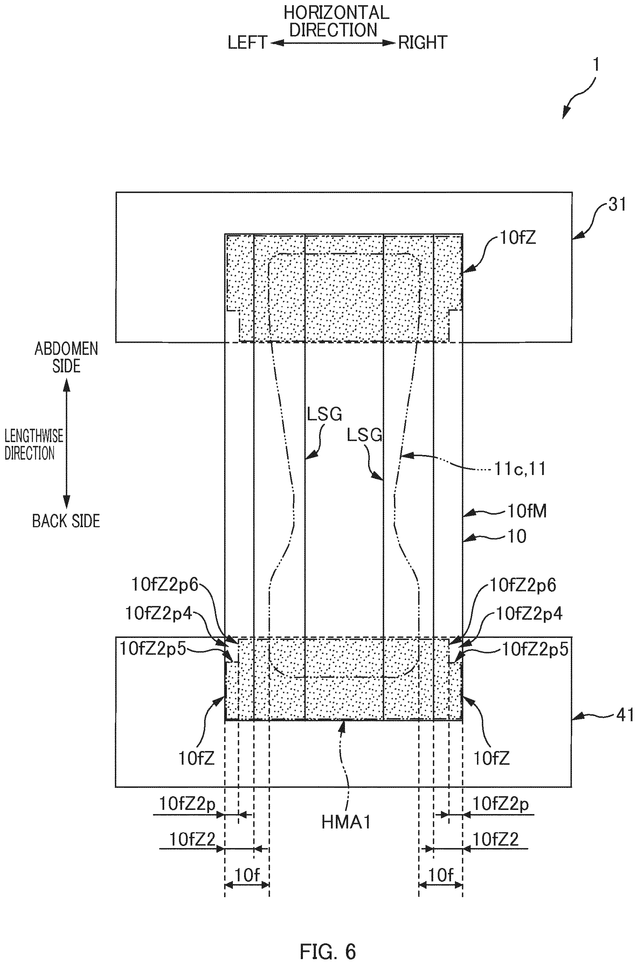

However, there is no limitation to such and, for example, the configuration can be the same as the above described abdomen side Z-shape folded portion 10fZ. In other words, as shown in Fig. 6, the above described predetermined portion 10fZ2p4 may be a non-fixed portion 10fZ2p4 that is not fixed to the back side waist part 41. And in such case, the portion 10fZ2p5 that is outwardly adjacent to the non-fixed portion 10fZ2p4 in the lengthwise direction in the above described portion 10fZ2p that extends out, and the portion 10fZ2p6 that is inwardly adjacent in the horizontal direction to the non-fixed portion 10fZ2p4 in the Z-shape folded portion 10fZ may respectively include fixed portions that are fixed to the back side waist part 41 with the adhesive HMA1. And when configured in such a manner, the leg openings LH can be made substantially large based on the former non-fixed portion 10fZ2p4, and the Z-shape folded portion 10fZ can be securely fixed to the back side waist part 41 based on the latter fixed portion.

Further, as shown in Figs. 3A, 3C, 3D and 3E, a barrier cuff LSG that can stand up from the skin side face is formed along the lengthwise direction to the skin side face of the flap part 10f in this example. The barrier cuff LSG has as the main body a sheet like member 15LSG extending along the lengthwise direction and the barrier cuff LSG is located horizontally inward with respect to the Z-shape folded portion 10fZ as a whole. And as shown in Fig. 3C, the horizontally outer end part 15LSGeW of the above described sheet like member 15LSG is fixed to the skin side face of the flap part 10f with adhesive HMA4, and hereby the extending portion 15LSGp that extends horizontally inward with respect to the adhesive HMA4 in this sheet like member 15LSG is made enabled to stand from the skin side face of the flap part 10f with the above described adhesive HMA4 as the support. In this example, this extending portion 15LSGp extends horizontally inward up to a location so to overlap with the absorbent core 11c. Additionally, the force for standing up is imparted by the elastic member 16 such as elastic strings. In other words, the tip end part that is located horizontally inward in the extending portion 15LSGp has an elastic member 16 fixed along the lengthwise direction in a state stretched in the lengthwise direction so to hereby impart to the extending portion 15LSGp a contraction force along the lengthwise direction. And with this contraction force, the extending portion 15LSGp is contracted along the lengthwise direction while forming a plurality of gathers so to have this extending portion 15LSGp stand up from the skin side face of the flap part 10f as the barrier cuff LSG, as a result.

As shown in Figs. 3A, 3C and 3D, the end parts 15LSGpe, 15LSGpe in the lengthwise direction of this extending portion 15LSGp are respectively fixed in states turned downward to the skin side face of the top sheet 13. In other words, these end parts 15LSGpe, 15LSGpe are laid over the skin side face of the top sheet 13 to be joined to this face with adhesive HMA5. And hereby, the extending portion 15LSGp is in a state maintained extending horizontally inward as a whole.

Further in this example, the location that becomes the above described support along the horizontal direction where the adhesive HMA4 is applied is set between the first folding location P1 and the second folding location P2 of the Z-shape folded portion 10fZ as shown in Fig. 3C, however, there is no limitation to such. For example, this location may be set horizontally inside the second folding location P2.

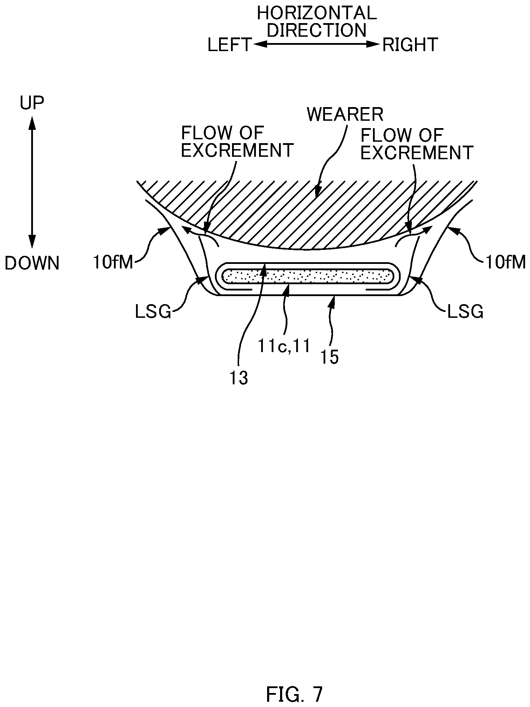

Further, since the aforementioned middle portion 10fM in the flap part 10f is a portion that is not fixed in a Z-folded state as mentioned above, this middle portion 10fM can be unfolded from the folded state to be spread out horizontally as shown in Fig. 3E, however, the maximum length L10fM from the above described support HMA4 to the horizontal tip end part 10fMe of the above described middle portion 10fM is longer the maximum length LLSG from this support HMA4 to the horizontal tip end part LSGe of the barrier cuff LSG when this middle portion 10fM is in a horizontally spread state. And when in such a state, the excrement that had leaked horizontally outward over the barrier cuff LSG can be held back by the above described middle portion 10fM, as shown in Fig. 7, and hereby the leg openings LH can achieve a further excellent leak prevention performance.

Further in this example, this barrier cuff LSG is also formed with the back sheet 15 similar to the flap part 10f. In other words, aforementioned sheet like member 15LSG (Fig. 3A) that forms the main body of the aforementioned barrier cuff LSG is formed with a part of the back sheet 15.

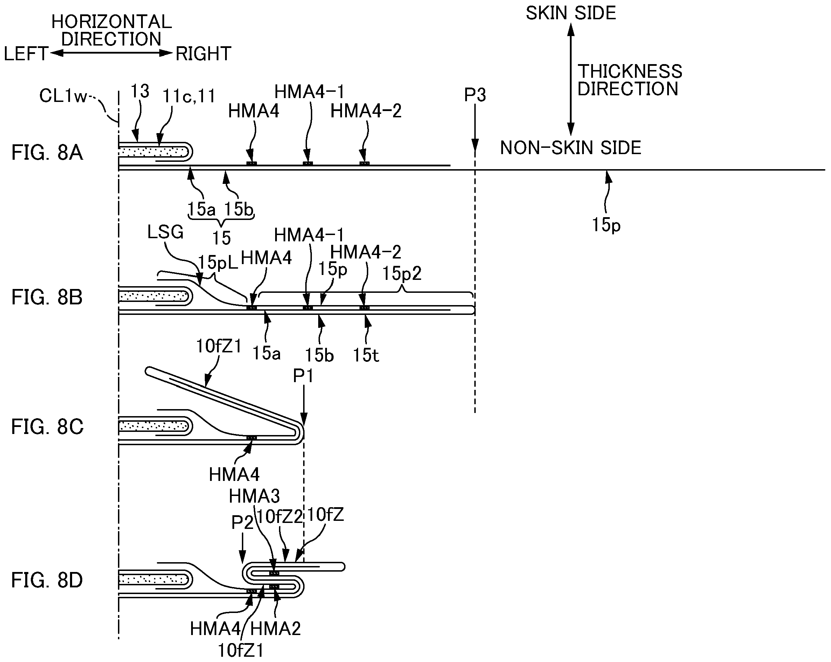

Figs. 8A to 8D are explanatory views illustrating the steps in which the barrier cuff LSG and a Z-shape folded portion 10fZ of the flap part 10f are formed from a back sheet 15. As has been already described, the back sheet 15 includes an outer covering sheet 15b and a leak prevention sheet 15a that is joined to the skin side face of the outer covering sheet 15b. Additionally, the outer covering sheet 15b is in a planar size such that the outer covering sheet 15b extends out from the leak prevention sheet 15a in both horizontal sides. Therefore, as shown in Figs. 8A and 8B, the portion 15p of the outer covering sheet 15b that extends horizontally outside from the leak prevention sheet 15a is firstly folded horizontally inward and toward the skin side at the folding location P3 where the leak prevention sheet 15a does not exist. And as shown in Fig. 8B, the portion 15p that is folded back is fixed to the skin side face of the leak prevention sheet 15a with adhesives HMA4, HMA4-1, HMA4-2 at a plurality of locations along the horizontal direction. Here, the adhesive HMA4 that is located at the innermost location along the horizontal direction among the adhesives HMA4, HMA4-1, HMA4-2 at the plurality of locations is the adhesive HMA4 that becomes the support of the aforementioned barrier cuff LSG. Therefore, the portion 15p1 that is at a location horizontally inside with respect to the above described adhesive HMA4 of the above described portion 15p that is folded back becomes the barrier cuff LSG.

Meanwhile, as shown in Fig. 8B, the portion 15p2 that is at a location horizontally outside with respect to the above described adhesive HMA4 of the portion 15 that is folded back forms a three layered structure sheet-like portion 15t by cooperating with both the leak prevention sheet 15a that overlaps this portion 15p2 and the outer covering sheet 15b that is located on the non-skin side of the leak prevention sheet 15a. In other words, this sheet-like portion 15t is in a three-layered structure made by sandwiching from the two faces of the leak prevention sheet 15a with the outer covering sheets 15b (15p2), 15b. And this three-layered structured sheet-like portion 15t is folded in the horizontal direction into a Z-shape to form the aforementioned Z-shape folded portion 10fZ as shown in Figs. 8C and 8D. In other words, the aforementioned inner fold part 10fZ1 is formed by this three-layered structured sheet-like portion 15t being folded back horizontally inward as well as toward the skin side at the aforementioned first folding location P1, as shown in Fig. 8C, and then the aforementioned outer fold part 10fZ2 is formed by the inner fold part 10fZ1 being folded back horizontally outward as well as toward the skin side at the aforementioned second folding location P2 as shown in Fig. 8D. Then the Z-shape folded portion 10fZ is formed in this folded back state by fixing with the aforementioned adhesives HMA2, HMA3.

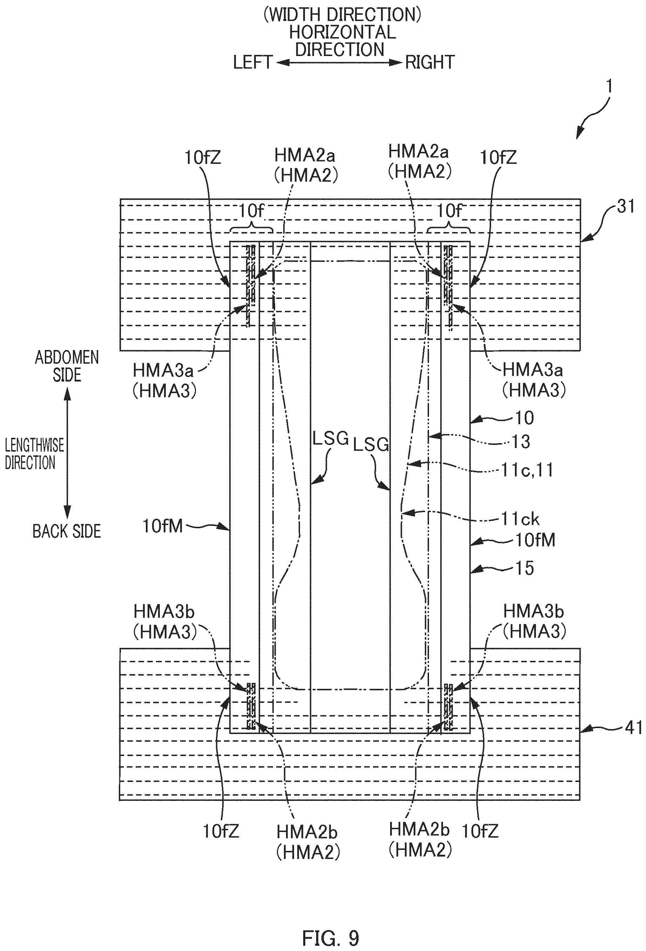

Fig. 9 is an explanatory view illustrating areas to which the adhesives HMA2, HMA3 are applied to the Z-shape folded portion 10fZ and is a schematic plan view of the spread-out state diaper 1 seen from the skin side.

As shown in this Fig. 9, both the adhesive HMA2 and the adhesive HMA3 are applied in a rectangular shape in plan view whose lengthwise side is longer that the horizontal side. Additionally, the former adhesive HMA2 is the adhesive HMA2 that joins the inner fold part 10fZ1 and the outer fold part 10fZ2 in the Z-shape folded portion 10fZ (Figs. 3C, 3D) as described above, however, these adhesives HMA2, HMA2 are also applied to the abdomen side and the back side since the Z-shape folded portions 10fZ, 10fZ are provided to each of the abdomen side and the back side being the two sides along the lengthwise direction. Meanwhile, the latter adhesive HMA3 is the adhesive HMA3 that joins the inner fold part 10fZ1 and the portion 10fk that is horizontally inside with respect to the first folding location P1 in the flap part 10f (Figs. 3C, 3D), however, this adhesive HMA3 is also provided to each of the abdomen side and the back side since each one of the Z-shape folded portions 10fZ, 10fZ are provided to the abdomen side and the back side. In the following, the adhesive HMA2 and the adhesive HMA3 that are located on the abdomen side along the lengthwise direction are also respectively called the "adhesive HMA2a" and the "adhesive HMA3a", and the adhesive HMA2 and the adhesive HMA3 that are located on the back side along the lengthwise direction are also respectively called the "adhesive HMA2b" and the "adhesive HMA3b."

Here, the abdomen side is the side that opposes the groin part of the wearer where this groin part is concave and is a portion that becomes horizontally narrowed when approaching from the abdomen toward the crotch. Therefore, a further improved fitting performance can be achieved with the flap part 10f folded to have a certain thickness and come into contact with the wearer's body compared to when horizontally spreading the flap part 10f that had been in a state folded on the abdomen side waist part 31 while extended horizontally outward with respect to the absorbent core 11c. On the other hand, the back side is the side that opposes the buttocks. And since the buttocks are in convex forms, the buttocks can be further effectively covered by horizontally spreading the flap part 10f and increasing the height of the flap part 10f.

Therefore in this example, both of the adhesives HMA2 and HMA3 have the lengthwise length of the abdomen side adhesive HMA2a (HMA3a) made longer than the lengthwise length of the back side adhesive HMA2b (HMA3b) as shown in Fig. 9. And hereby, the abdomen side has the area that comes along the wearer's body in the folded state long by elongating the length of adhesive HMA2a (HMA3a) along the lengthwise direction so that an improved fitting performance is achieved at the groin part, while the back side has an improved performance in covering the buttocks by shortening the length of adhesive HMA2b (HMA3b) in the lengthwise direction to allow the flap part 10f to spread easily in the horizontal direction.

As shown in Fig. 9, when the lengthwise lengths of these adhesives HMA2, HMA3 are compared, between the adhesives HMA2a and HMA3a that are located on the abdomen side, the latter adhesive HMA3a is provided longer than the former adhesive HMA2a. Therefore, the flap part 10f can be made to come into contact with the groin part with excellent fitting performance while allowing the folded state to be easily maintained. In other words, the portion that extends outward outside in the horizontal direction with respect to the absorbent core 11c in the flap part 10f can be easily maintained in a folded state on the abdomen side waist part 31 by making the length of the adhesive HMA3a relatively long. Meanwhile, the portion 10fZ2pa (Fig. 5) that is located horizontally outside with respect to the adhesive HMA2a in the outer fold part 10fZ2 is made to stand easily by making the length of the adhesive HMA2a relatively short, and hereby the fitting performance of the flap part 10f to the groin part can be improved.

By the way, in the present embodiment, four types of elastic members 17, 18, 19, 20 are arranged side by side in the horizontal direction (Fig. 3C) in order to impart lengthwise contraction force to the absorbent main body 10 or the flap part 10f thereof. In the following, description of these four types of elastic members 17, 18, 19, 20 is given. All of the elastic members 17, 18, 19, 20 are stretched in the lengthwise direction and fixed with adhesives (not shown) to the absorbent main body 10 or the flap part 10f thereof, and hereby a contraction force is imparted to the absorbent main body 10 or the flap part 10f. Additionally, in this example, elastic strings are used for these four types of elastic members 17, 18, 19, 20, however, there is no limitation to such.

Firstly, when the spread-out state diaper 1 in Fig. 3C is seen from the skin side, the type one elastic member 17 is arranged to match the horizontal location of the support HMA4 of the barrier cuff LSG, at the flap part 10f. Therefore, this elastic member 17 can impart a contraction force along the lengthwise direction at the support HMA4 of the barrier cuff LSG. And hereby, the support HMA4 can lift up the absorbent main body 10 so to come close to the wearer's skin.

In this example, the type one elastic member 17 is inserted between the outer covering sheet 15b and the leak prevention sheet 15a of the back sheet 15, however there is no limitation to such. In other words, the type one elastic member 17 may be fixed to the skin side face or the non-skin side face of the back sheet 15 with adhesive (not shown.) This applies in the same manner to the type two to type four elastic members 18, 19, 20 that are described in the following.

In this example, the location of the above described support HMA4 is set horizontally outside the absorbent core 11c, that is, this support HMA4 is appropriately separated horizontally outside from the wearer's crotch. And hereby, the barrier cuff LSG is made to come into contact with the wearer's skin from a location further horizontally outward. And as a result, crushing of the barrier cuff LSG that is likely to occur when the barrier cuff LSG is located horizontally inside can be effectively averted and as a result, the barrier cuff LSG can be easily maintained in a standing state.

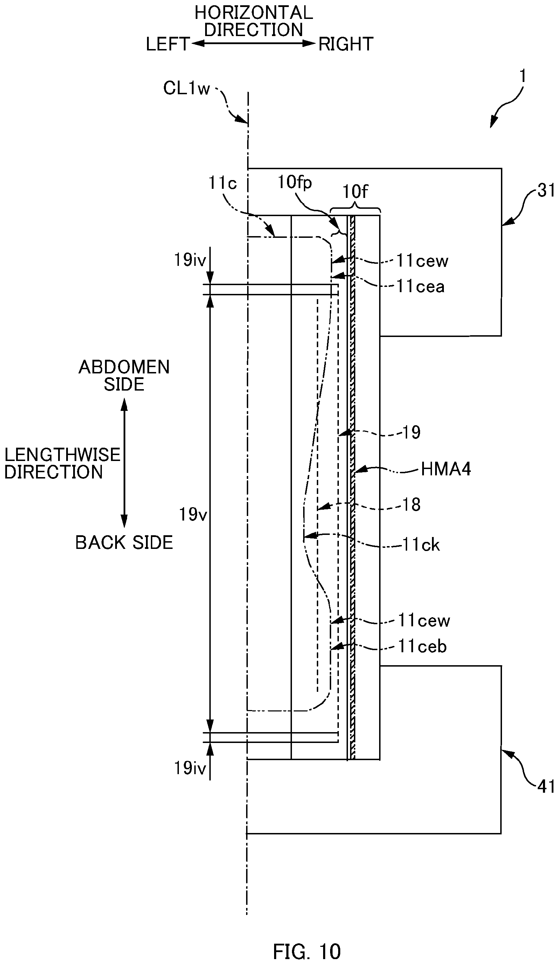

Next, the type two elastic member 18 (corresponding to the first elastic member) is arranged to overlap with the absorbent core 11c at the two lengthwise end parts 11cea, 11ceb of the absorbent core 11c but does not overlap with the absorbent core 11c at the constricted part 11ck of this absorbent core which is located between the two end parts 11cea, 11ceb, when the diaper 1 in an spread-out state and a pattern state of Fig. 10 (Fig. 3C to 3E) is seen from the skin side. Therefore, the under-pants type diaper 1 when in a worn state can have the absorbent main body 10 kept from excessively drooping downward. In other words, the portion where the above described constricted part 11ck is located and the portions proximate thereto in the absorbent main body 10 is commonly most likely to droop downward, however, this type two elastic member 18 is located to a position relatively close to the constricted part 11ck according to the above described positioning. Therefore, a contraction force can be effectively imparted to the constricted part 11ck through the flap part 10f. And hereby, the constricted part 11ck can be lifted up to come close to the wearer's skin with this contraction force so that the absorbent main body 10 is kept from drooping excessively downward. As a result, a gap being created between the wearer and the absorbent main body 10 can be suppressed so that the overall leak prevention performance of the diaper 1 can be improved.

Subsequently, the type three elastic member 19 (corresponding to the second elastic member) is provided to the portion 10fp between the support HMA4 of the barrier cuff LSG in the flap part 10f and the horizontally outermost end 11cew of the absorbent core 11c when the diaper 1 in an spread-out state and a pattern state of Fig. 10 (Fig. 3C to 3E) is seen from the skin side. Therefore, this type three elastic member 19 being located at a position relatively close to the absorbent core 11c can effectively impart a contraction force to this core 11c through the flap part 10f. And hereby, even when the absorbent core 11c absorbs excrement and gets heavy after the wearer puts on the pants-type diaper 1, the absorbent core 11c can be lifted up to come close to the wearer's skin with the above described contraction force.

Further, when this type three elastic member 19 is seen with respect to the lengthwise direction with reference to Fig. 10, this type three elastic member 19 includes an effective portion 19v that can impart a contraction force and ineffective portions 19iv, 19iv that cannot impart a contraction force and that are located to the two lengthwise sides of the effective portion 19v. In other words, the lengthwise end parts 19iv, 19iv of the type three elastic member 19 are not fixed to the flap part 10f with adhesives and the like and are in a state contracted in the lengthwise direction up to a natural length as well, so that such are ineffective portions 19iv, 19iv that cannot impart a contraction force to the flap part 10f.

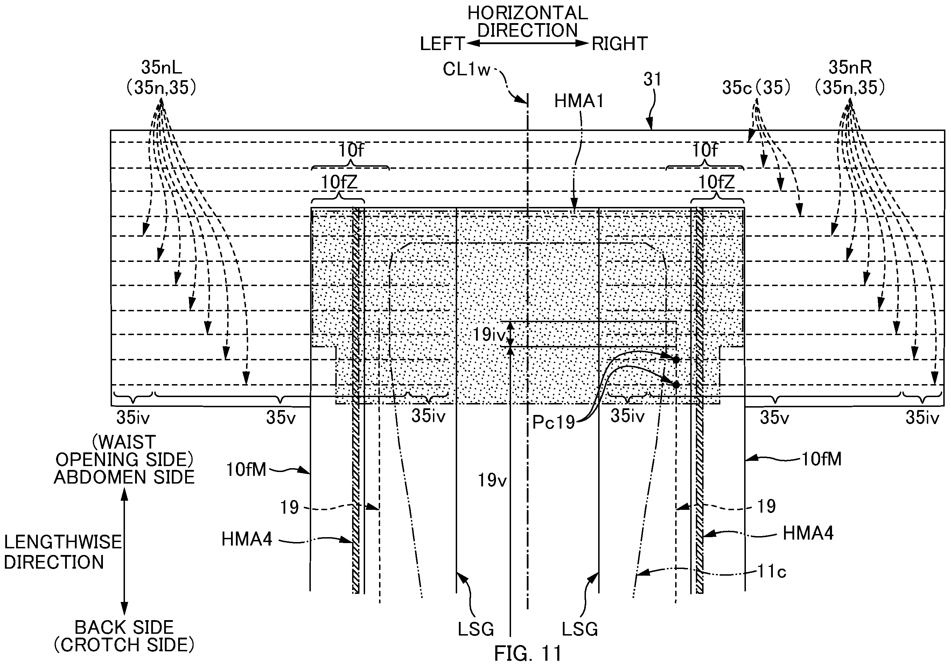

Meanwhile, as described above, the abdomen side waist part 31 in Fig. 11 includes a plurality of abdomen side elastic members 35, 35 ... for imparting horizontal contraction force to this abdomen side waist part 31. And the left side elastic member 35nL and the right side elastic member 35nR of the discontinuous elastic member 35n among these abdomen side elastic members 35, 35 ... each include an effective portion 35v that can impart contraction force and ineffective portions 35iv, 35iv that are located on the two horizontal sides of the effective portion 35v and cannot impart a contraction force. In other words, both elastic members being the left side elastic member 35nL and the right side elastic member 35nR do not have the horizontal end parts 35iv, 35iv fixed to the flap part 10f with adhesives and the like and are in a state contracted in the lengthwise direction up to a natural length as well, so that they are ineffective portions 35iv that cannot impart a contraction force to the flap part 10f.

Further, the left side elastic member 35nL and the right side elastic member 35nR are in a right-left mirror image relation with each other. And the other points are substantially the same. Therefore, the following describes only the right side elastic member 35nR representing the two, as mentioned above.

It is preferable that the intersecting location Pc19 overlaps the area (dotted area in Fig. 11) is joined to the abdomen side waist part 31 in the flap part 10f with adhesive HMA1, the intersecting location Pc19 being where, when the spread-out state diaper 1 in Fig. 11 is seen from the skin side, the effective portion 35v of at least one right side elastic member 35nR among the plurality of the right side elastic members 35nR, 35nR ... that are arranged in the lengthwise direction of the diaper 1 intersects the effective portion 19v of the type three elastic member 19. For example, the effective portion 35v of a total of two right side elastic members 35nR, when counting from the crotch side, satisfies the above in this example.

And when configured in the above manner, the contraction force of the right side elastic member 35nR can be quickly transferred to the effective portion 19v of the type three elastic member 19 through the abdomen side waist part 31 and the flap part 10f. Therefore, the contraction force of this right side elastic member 35nR can also effectively contribute to the lifting up of the absorbent main body 10 toward the skin side performed through the flap part 10f that is brought about by the contraction force of the type three elastic member 19. And hereby, the absorbent main body 10 can be further effectively kept from drooping.

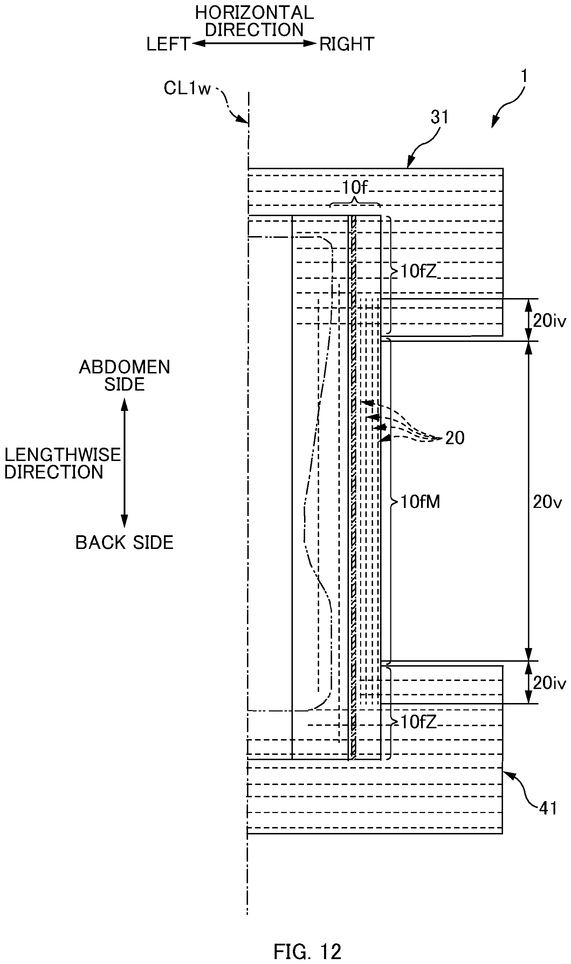

Fig. 12 is a schematic plan view of the type four elastic member 20 (corresponding to the third elastic member.) The type four elastic member 20 is provided to the flap part 10f in a manner extending across in the lengthwise direction between the abdomen side Z-shape folded portion 10fZ and the back side Z-shape folded portion 10fZ. And in this example, four of these type four elastic members 20 are provided to the outer fold part 10fZ2 of the Z-shape folded portion 10fZ and a single type four elastic member 20 is provided to the inner fold part 10fZ1 as shown in Figs. 3C and 3D. Further as shown in Fig. 12, this type four elastic member 20 includes an effective portion 20v that can impart a contraction fore and ineffective portions 20iv, 20iv that cannot impart a contraction force and are located on the two lengthwise sides of the effective portion 20v. In other words, the lengthwise end parts 20iv, 20iv of the type four elastic member 20 is not fixed to the Z-shape folded portion 10fZ with adhesives and the like and is in a state contracted in the lengthwise direction up to a natural length as well, so that such are ineffective portions 20iv that cannot impart a contraction force to the flap part 10f.

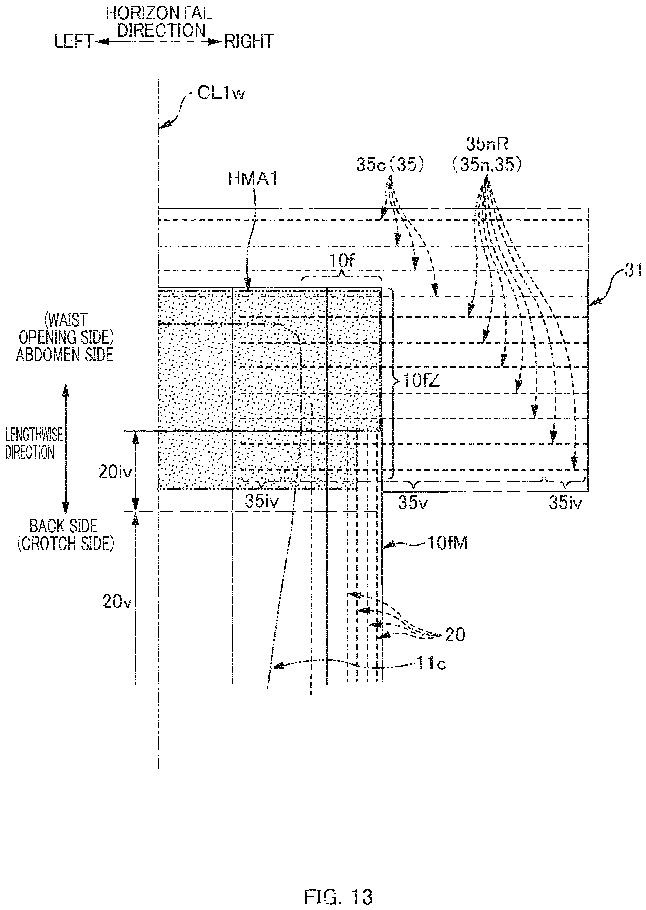

Meanwhile, as shown in Fig. 13, the right side elastic member 35nR with respect to the discontinuous elastic member 35n of the abdomen side waist part 31 also includes an effective portion 35v that can impart a contraction force and ineffective portions 35iv, 35iv that cannot impart a contraction force and are located on the two horizontal sides of the effective portion 35v.

It is preferable that the effective portion 35v of at least a single right side elastic member 35nR among the plurality of the right side elastic members 35nR, 35nR ... that are arranged in the lengthwise direction of the diaper 1 does not intersect the effective portion 20v of the type four elastic member 20 at the area (dotted area in Fig. 13) where the abdomen side waist part 31 is joined with adhesive HMA1 in the flap part 10f when the opened and the pattern state diaper 1 in Fig. 13 is seen from the skin side. For example, the effective portion 20v of the type four elastic member 20 is located inside in the lengthwise direction with respect to the position of the total of seven right side elastic members 35nR, 35nR that located on the crotch side along the lengthwise direction, and hereby the effective portion 20v of the type four elastic member 20 does not intersect with the effective portions 35v, 35v of all the seven right side elastic members 35nR, 35nR.

And when configured in the above manner, the flap part 10f allows the leg openings LH to be easily maintained in states that enhance the legs of the wearer to pass through when the wearer puts on this pants-type diaper 1. In other words, when the abdomen side waist part 31 and the back side waist part 41 of the diaper 1 are pulled to the two horizontal sides in order for the wearer to put on the underpants-type diaper 1, this pulling force is also transmitted to the right side elastic member 35nR of the abdomen side waist part 31. However, in this occasion, the effective portions 35v, 20v do not intersect with each other in the area (the dotted area in Fig. 13) where the abdomen side waist part 31 is joined in the flap part 10f thereby making it difficult for the force to be transmitted of between the right side elastic member 35nR and the type four elastic member 20. Therefore, almost none of the above pulling force is transmitted from the right side elastic member 35nR to the type four elastic member 20. For such reason, the portion proximate the Z-shape folded portion 10fZ in the middle portion 10fM of the flap part 10f is suppressed from spreading horizontally outward due to the above described pulling force. And hereby, the leg openings LH can be kept from becoming substantially small due to this proximate portion spreading horizontally outward. And as a result, the leg openings LH are easily maintained in states that enhance the legs of the wearer to pass through when the wearer puts on this pants-type diaper 1.

Further in this example, since the Z-shape folded portion 10fZ is formed with the back sheet 15 according to the aforementioned procedure shown in Figs. 8A to 8D, the leak prevention sheet 15a (corresponding to the liquid impermeable sheet) of the back sheet 15 is arranged also to extend horizontally with respect to both the inner fold part 10fZ1 and the outer fold part 10fZ2, as shown in Fig. 3C. And hereby, the leak prevention performance of the inner fold part 10fZ1 and the outer fold part 10fZ2 is enhanced. And along with this, the aforementioned middle portion 10fM that is located between the abdomen side Z-shape folded portion 10fZ and the back side Z-shape folded portion 10fZ also has the leak prevention sheet 15a extending up to the same horizontal location described above, as shown in Fig. 3E. Therefore, this middle portion 10fM also has the leak prevention performance enhanced similar to the aforementioned outer fold part 10fZ2 and the inner fold part 10fZ1.

However in this example, as shown in Fig. 3C, the horizontal tip end part 10fZ2e in the outer fold part 10fZ2 is a non-positioned portion where the leak prevention sheet 15a is not placed and this non-positioned portion has the above described type four elastic member 20 (corresponding to the fifth elastic member) provided. And this is similar to the above described middle portion 10fM shown in Fig. 3E. Therefore, an uncomfortable touch by the skin with this tip end part 10fe of the flap part 10f caused by the existence of the leak prevention sheet 15a can be averted. Further, this tip end part 10fe can come in intimate contact with the wearer' s leg by the contraction force of the type four elastic member 20 and this also effectively contributes to improve the leak prevention performance of the leg openings LH.

Further, as aforementioned, the portion 10fMZ1 (Fig. 3E) (hereinafter called middle portion inner fold part 10fMZ1) that is located between the two lengthwise inner fold parts 10fZ1, 10fZ1 at the middle portion 10fM (Fig. 3A) of the flap part 10f is imparted a contraction force in the lengthwise direction based on the above described type four elastic member 20a (20) (corresponding to the inner fold part elastic member) that is provided to the inner fold part 10fZ1, and similarly, the portion 10fMZ2 (Fig. 3E) (hereinafter called middle portion outer fold part 10fMZ2), at this middle portion 10fM, which is located between the two lengthwise outer fold parts 10fZ2, 10fZ2 is imparted a contraction force in the lengthwise direction based on the above described type four elastic member 20b (20) that is provided to the outer fold part 10fZ2. Here, it is preferable that the magnitude of the lengthwise contraction force (N/cm) per unit horizontal length imparted to the former middle portion inner fold part 10fMZ1 is greater than the magnitude of the lengthwise contraction force (N/cm) per unit horizontal length imparted to the latter middle portion outer fold part 10fMZ2 when the flap part 10f is under a 70% stretched state, where the 100% stretched state is a state when the flap part 10f is stretched until there is no wrinkles formed in the lengthwise direction.

And when configured in the above manner, the middle portion inner fold part 10fMZ1 contracts greater in the lengthwise direction compared to the middle portion outer fold part 10fMZ2 when the middle portion 10fM of the flap part 10f stands when the diaper 1 is worn so that the middle portion outer fold part 10fMZ2 is in a state slightly outwardly slanted, as shown in Fig. 3E. And this together with the Z-fold structure that can spread horizontally outward makes the middle portion outer fold part 10fMZ2 come into contact with the wearer's body with the face in a spread state.

By the way, the above described contraction forces (N/cm) of the middle portion inner fold part 10fMZ1 and the middle portion outer fold part 10fMZ2 are measured in the following manner.

Firstly, the middle portion 10fM (Fig. 3A) of the flap part 10f is cut at the first folding location P1 (Fig. 3E) along the lengthwise direction and together, the abdomen side and the back side Z-shape folded portions 10fZ, 10fZ in the lengthwise direction are cut off from the middle portion 10fM of the flap part 10f. And hereby, the horizontal tip end side portion of this middle portion 10fM (Fig. 3E) is cut and separated from the diaper 1. Further, this tip end side portion that has been cut off is further cut along the second fold back location P2 (Fig. 3C) in the lengthwise direction to thereby create a sample of the above described middle portion inner fold part 10fMZ1 and a sample of the above described middle portion outer fold part 10fMZ2 from this tip end side portion.

Then using 10mm of the lengthwise end parts of the respective samples as the holding parts, and when the portions beside the holding parts are regarded as the remaining portions, the lengthwise dimension L1 (mm) and the horizontal dimension L2 (mm) of the samples are measured in advance with the remaining portion in states spread in the lengthwise and the horizontal directions in a manner having wrinkles removed.

Thereafter, the holding parts of the samples are held by the upper and lower chucks equipped to the tensile tester (e.g., Autograph AGS-X etc. made by Shimadzu Corporation) and then the upper and the lower chucks have the distance therebetween widened so that the sample in the contracted state is stretched in the lengthwise direction until 85% of the length of the above described L1 is reached at a tension rate of 100 (mm/min) and the distance between the chucks is returned to a narrowed state until 70% of L1 is reached after a certain time. And the contraction force (N) in this narrowed state is recorded for the contraction force (N) to be converted using the L2 dimension (mm) to the contraction force per unit horizontal length (10mm). Then this contraction force (N/cm) after conversion is regarded as the above described lengthwise contraction force (N/cm) per unit horizontal length.

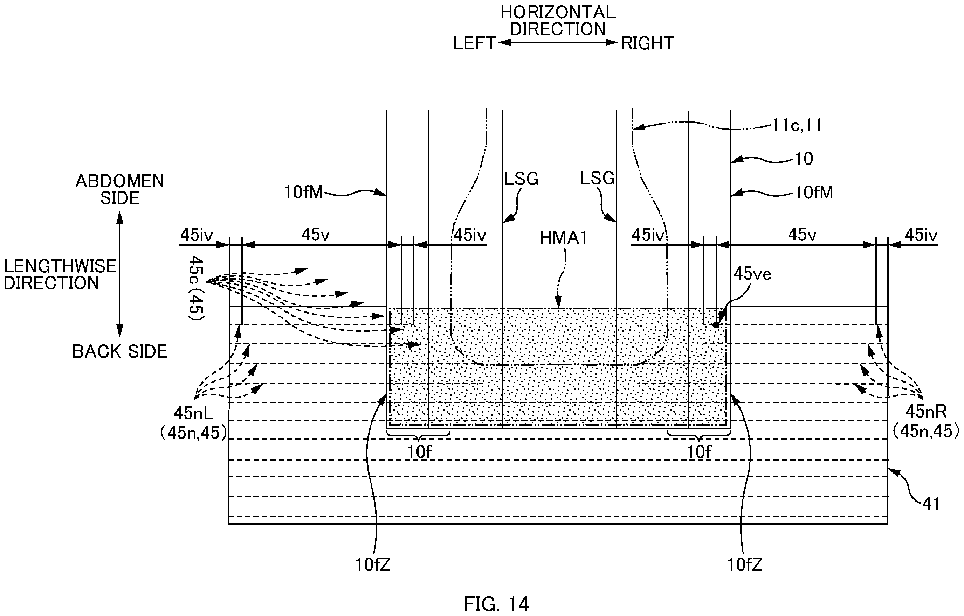

Fig. 14 is a schematic enlarged plan view of the back side waist part 41. Here, Fig. 14 shows the underpants-type diaper 1 in a pattern state.

As mentioned above, this back side waist part 41 includes a plurality of back side elastic members 45 for imparting a horizontal contraction force to this back side waist part 41. And the left side elastic member 45nL and the right side elastic member 45nR of the discontinuous elastic member 45n among these back side elastic members 45, 45 ... respectively include the effective portions 45v that can impart contraction force and the portions 45iv, 45iv that are located on the two sides along the horizontal direction of the effective portion 45v and cannot impart contraction force. In other words, both the left side elastic member 45nL and the right side elastic member 45nR do not have the horizontal end parts 45iv, 45iv fixed to the flap part 10f with adhesives and the like and simultaneously are in a state contracted in the lengthwise direction to a natural length. Hereby, the respective parts are ineffective parts 45iv, 45iv that cannot impart a contraction force to the flap part 10f.

Further, the left side elastic member 45nL and the right side elastic member 45nR are in a right-left mirror image relation with each other and besides this, the other points are substantially the same. Therefore, the following describes only the right side elastic member 45nR representing the two.

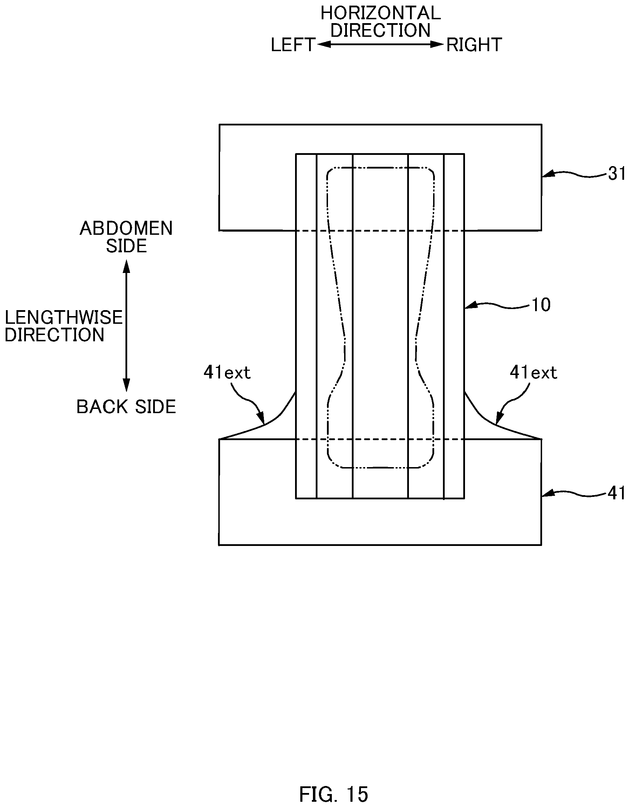

It is preferable that at least a part of the effective portion 45v of at least a single right side elastic member 45nR among the right side elastic members 45nR, 45nR is located to an area (dotted area in Fig. 14 which is also called the joined area in the following) that has the Z-shape folded portion 10fZ of the flap part 10f joined with adhesive HMA1 in the back side waist part 41. And in this example, the left end part 45ve of each effective portion 45v of all the right side elastic members 45nR, 45nR ... among all four right side elastic members 45nR, 45nR ... is located at the above described joined area. Therefore, when the abdomen side waist part 31 and the back side waist part 41 of the diaper 1 is pulled horizontally in both sides in order for the wearer to put on the underpants-type diaper 1, this pulling force is transmitted to the Z-shape folded portion 10fZ through the effective portions 45v of the above described right side elastic members 45nR and hereby, the portions proximate the Z-shape folded portion 10fZ in the middle portion 10fM of the flap part 10f is spread out in the horizontal direction. And the buttocks of the wearer can be quickly covered with the spread out portions, as a result. Hereby, the portion on the crotch side in the back side waist part 41 does not need to have extended out parts 41ext, 41ext such as those shown in Fig. 15 provided for covering the buttocks.

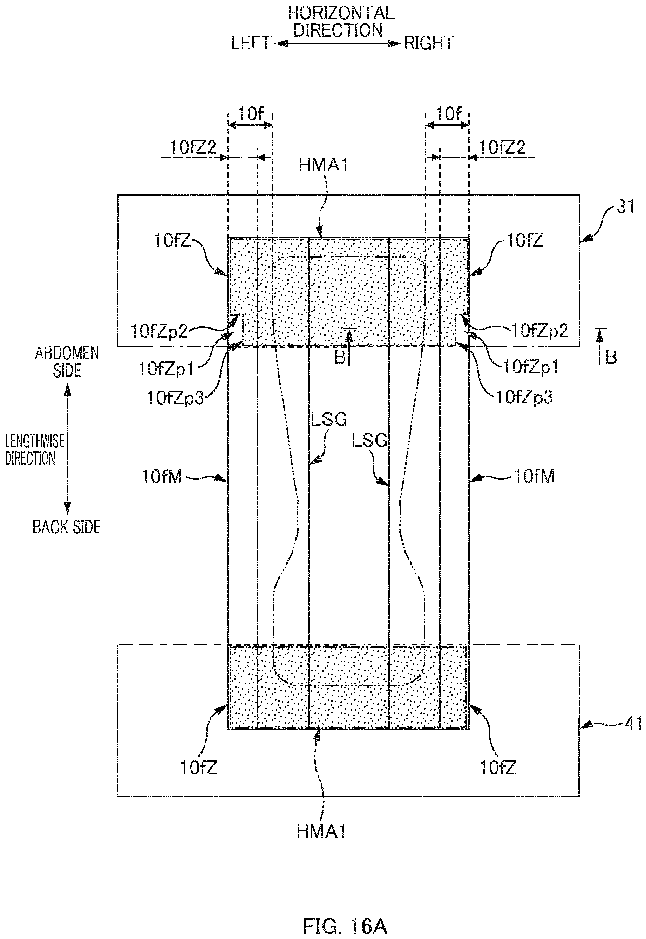

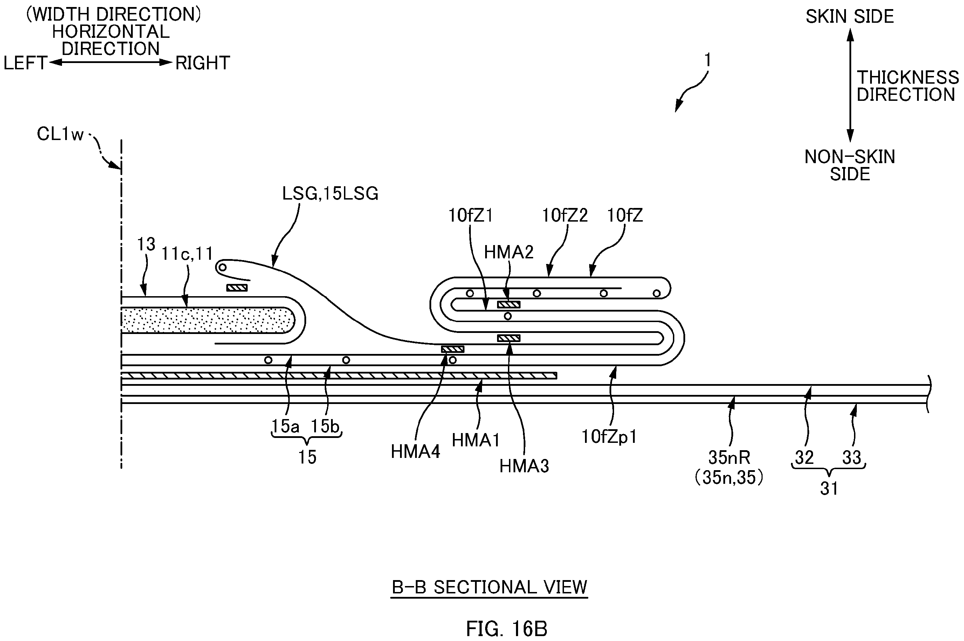

Figs. 16A and 16B are explanatory views illustrating modified examples of the Z-shape folded portion 10fZ. Fig. 16A is a schematic plan view of the spread-out state diaper 1 seen from the skin side and Fig. 16B is a sectional view taken along line B-B in Fig. 16A.

The aforementioned embodiment included to the outer fold part 10fZ2 of the Z-shape folded portion 10fZ, a portion 10fZ2p that extends horizontally outward with respect to the inner fold part 10fZ1 as shown in Figs. 4 and 5. And a non-fixed portion 10fZ2p1 that is not fixed to the abdomen side waist part 31 with adhesive HMA1 being provided to this extending portion 10fZ2p allowed the leg portion to be substantially large, however, the above described extending portion 10fZ2p is not included in this modified example, as shown in Fig. 16B. In other words, this modified example has the outer fold part 10fZ2 slightly withdrawn horizontally inward with respect to the inner fold part 10fZ1, that is, the outer fold part 10fZ2 does not extend outward.

However, when a non-fixed portion 10fZp1 is provided in the following manner, similar to the aforementioned embodiment, an effect of enlarging the leg openings LH can be achieved even in such a modified example.

In other words, as shown in Figs. 16A and 16B, the predetermined portion 10fZp1 including the horizontal outer edge part in the Z-shape folded portion 10fZ, being the above described predetermined portion 10fZp1 including the portion 10fZp1 that is on the innermost side in the lengthwise direction of the portion that overlaps the abdomen side waist part 31 is regarded as the non-fixed portion 10fZp1 that is not fixed to the abdomen side waist part 31. And in this way, this not-fixed portion 10fZp1 can be separated from the abdomen side waist part 31 so to thereby allow the leg openings LH to be substantially enlarged by the dimension of the portion 10fZp1.

By the way, similar to the aforementioned embodiment, this modified example also can include a fixed portion adjacent the non-fixed portion 10fZp1. In other words, as shown in Fig. 16A, the portion 10fZp2 that is outwardly adjacent along the lengthwise direction to the above described non-fixed portion 10fZp1 in the Z-shape folded portion 10fZ and the portion 10fZp3 that is inwardly adjacent along the horizontal direction to the non-fixed portion 10fZp1 in the Z-shape folded portion 10fZ may be respectively fixed to the abdomen side waist part 31 with adhesives HMA1. And in this way, the Z-shape folded portion 10fZ can be securely fixed to the abdomen side waist part 31.

### ===Other Embodiments===

The embodiments of the present invention has been described, however, the foregoing embodiments are intended to facilitate the understanding of the present invention but not to limit the invention. And it is needless to say that modifications and improvements of the present invention are possible without departing from the effect of the invention, and equivalents thereof are also encompassed by the invention. For example, the following modifications are possible.

In the aforementioned embodiment, the Z-shape folded portion 10fZ was made by folding the flap part 10f two times in the horizontal direction, however, there is no limitation to such. In other words, as long as the flap part 10f includes a portion that is fixed in a state folded in a Z-shape, such are included in the concept of the present invention. For example, a portion that is folded and fixed in a W-shape that has the above described Z-shape folded a further additional time is also included in the concept of the present invention since this includes a portion that is folded and fixed in a Z-shape. Similarly, it is needless to say that cases where the number of times of the folding is greater being four or more is also included in the concept of the present invention.

In the aforementioned embodiment, the flap part 10f was formed with the back sheet 15 as shown in Fig. 3C, however there is not limitation to such and the flap part 10f may be formed with a sheet different from the back sheet 15. For example, a different sheet that forms the flap part 10f may be provided to the respective horizontal sides of the back sheet 15. Similarly, in the aforementioned embodiment, the barrier cuff LSG was also formed with the back sheet 15, however, there is no limitation to such. In other words, the barrier cuff LSG may be formed with a sheet different from the back sheet 15.

### [Reference Signs List]

1 disposable diaper, 10 absorbent main body, 10ea end part, 10eb end part, 10f flap part, 10fM middle portion, 10fMe tip end part, 10fMZ1 middle portion inner fold part, 10fMZ2 middle portion outer fold part, 10fZ Z-shape folded portion, 10fZ1 inner fold part, 10fZ2 outer fold part, 10fZ2p1 non-fixed portion, 10fZ2e tip end part, 10fZ2p extending portion, 10fZ2p1 predetermined portion, 10fZ2p2 adjacent portion, 10fZ2p3 adjacent portion, 10fZ2p4 predetermined portion, 10fZ2p5 adjacent portion, 10fZ2p6 adjacent portion, 10fZ2pa portion, 10fZk overlapping portion, 10fZp1 predetermined portion, 10fZp2 adjacent portion, 10fZp3 adjacent portion, 10fe tip end part, 10fk portion, 10fp portion, 11 absorbent body, 11c absorbent core, 11cea end part, 11ceb end part, 11cew outermost end, 11ck constricted part, 11e end part, 13 top sheet, 13e end part, 15 back sheet, 15LSG sheet like member, 15LSGew outer end part, 15LSGp extending portion, 15LSGpe end part, 15a leak prevention sheet (liquid impermeable sheet), 15b outer covering sheet, 15pw portion extending out, 15p portion extending out (folded back portion), 15p1 portion, 15p2 portion, 15t sheet-like portion, 16 elastic member, 17 type one elastic member, 18 type two elastic member (first elastic member), 19 type three elastic member (second elastic member), 19v effective portion, 19iv end part (ineffective portion), 20 type four elastic member (third elastic member), 20a type four elastic member (third elastic member, elastic member for inner fold part), 20b type four elastic member (third elastic member, elastic member for outer fold part), 20v effective portion, 20iv end part (ineffective portion), 31 abdomen side waist part, 31e end part, 32 nonwoven fabric, 33 nonwoven fabric, 35 abdomen side elastic member, 35c continuous elastic member, 35n discontinuous elastic member, 35nL left side elastic member, 35nLeL left end part, 35nLeR right end part, 35nR right side elastic member, 35nReL left end part, 35nReR right end part, 35v effective portion, 35iv end part (ineffective portion), 41 back side waist part, 41e end part, 41ext extended out part, 42 nonwoven fabric, 43 nonwoven fabric, 45 back side elastic member, 45c continuous elastic member, 45n discontinuous elastic member, 45nL left side elastic member, 45nLeL left end part, 45nLeR right end part, 45nR right side elastic member, 45nReL left end part, 45nReR right end part, 45v effective portion, 45iv end part (ineffective portion), 45ve left end part, BH waist opening, LH leg opening, P1 first folding location, P2 second folding location, P3 folding location, CL1 center location, CLlw center location, CL10 predetermined location, LSG barrier cuff, HMA1 adhesive, HMA2 adhesive, HMA3 adhesive, HMA4 adhesive (support), HMA4-1 adhesive, HMA4-2 adhesive, HMA5 adhesive, LSGe tip end part, Pc19 intersecting location,

## Claims

1. An underpants-type disposable diaper (1) having a vertical direction and a horizontal direction that intersect each other, comprising:
an absorbent main body (10) having a lengthwise direction and being folded in two at a predetermined location (CL10) in the lengthwise direction as well as having the lengthwise direction arranged along the vertical direction;
an abdomen side waist part (31) overlapping and being joined to a non-skin side face of an end part on one side in the lengthwise direction of the absorbent main body; and
a back side waist part (41) overlapping and being joined to a non-skin side face of an end part on another side in the lengthwise direction of the absorbent main body; wherein
the absorbent main body (10) includes an absorbent core (11c) that absorbs liquid and a sheet-like flap part (10f) that extends horizontally outward with respect to the absorbent core,
the flap part (10f) includes on each of the one side and the other side in the lengthwise direction, a Z-shape folded portion that is fixed in a state folded along the horizontal direction in a Z-shape,
portions located horizontally outward with respect to the absorbent core (11c) in the abdomen side waist part (31) and the back side waist part (41) respectively have the Z-shape folded portions (10fZ) of the flap part fixed,
the diaper further comprises a barrier cuff (LSG) that can stand from a skin side face and is supported by a support (HMA4) that is provided to the skin side face of the flap part (10f),
the flap part includes a middle portion (10fM) that is not fixed in a folded state at a location between the Z-shape folded portion (10fZ) on the one side and the Z-shape folded portion (10fZ) on the other side, and
a maximum length (L10fM) from the support (HMA4)to a horizontal tip end part (10fMe) of the middle portion, when the middle portion is unfolded and in a horizontally spread state, is greater than a maximum length (LLSG) from the support (HMA4) to a horizontal tip end part (LSGe) of the barrier cuff.

2. An underpants-type disposable diaper according to claim 1, wherein
a predetermined portion (10fZ2p1) includes a horizontal outer edge part in the Z-shape folded portion (10fZ), the predetermined portion including an innermost portion in the lengthwise direction of a portion that overlaps the abdomen side waist part (31) is a non-fixed portion that is not fixed to the abdomen side waist part,
a portion (10fZ2p3) inwardly adjacent to the non-fixed portion (10fZ2p1) in the horizontal direction in the Z-shape folded portion includes a fixed portion that is to be fixed to the abdomen side waist part, and
a portion (10fZ2p2) outwardly adjacent to the non-fixed portion (10fZ2p1) in the lengthwise direction in the Z-shape folded portion includes a fixed portion that is to be fixed to the abdomen side waist part (31).

3. An underpants-type disposable diaper according to claim 1 or 2, wherein
the Z-shape folded portion (10fZ) includes an inner fold part (10fZ1) that is folded back horizontally inward at a first folding location (P1) in the horizontal direction to extend horizontally inward with respect to the first folding location (P1) and an outer fold part (10fZ2) that is folded back horizontally outward at a second folding location (P2) in the inner fold part to extend horizontally outward with respect to the second folding location (P2),
the inner fold part (10fZ1) and the outer fold part (10fZ2) have faces opposing each other joined, and a horizontally inner portion (10fk) with respect to the first folding location (P1) in the flap part (10f) and the inner fold part (10fZ1) have faces opposing each other joined as well, in the Z-shape folded portion, and
the outer fold part (10fZ2) has a portion (10fZ2p) extending horizontally outward with respect to the inner fold part (10fZ1) .

4. An underpants-type disposable diaper according to claim 3, wherein
a predetermined portion (10fZ2p1) that includes a horizontal outer edge part of the extending portion (10fZ2p) in the outer fold part and that further includes an innermost portion in the lengthwise direction of a portion that overlaps the abdomen side waist part (31) is a non-fixed portion that is not fixed to the abdomen side waist part,
a portion (10fZ2p2) that is outwardly adjacent to the non-fixed portion (10fZ2p1) in the lengthwise direction in the extending portion (10fZ2p) in the outer fold part (10fZ2) includes a fixed portion that is to be fixed to the abdomen side waist part (31), and
a portion (10fZ2p3) that is inwardly adjacent in the horizontal direction to the non-fixed portion (10fZ2p1) in the Z-shape folded portion (10fZ) includes a fixed portion that is to be fixed to the abdomen side waist part (31).

5. An underpants-type disposable diaper according to any one of claims 1 to 4, wherein
the absorbent core (11c) includes, a constricted part (11ck) that has a small horizontal dimension with respect to two end parts (11cea, 11ceb) in the lengthwise direction of the absorbent core, at a location between the two end parts in the lengthwise direction and
the absorbent main body (10) includes a first elastic member (18) along the lengthwise direction to impart to the absorbent main body a contraction force in the lengthwise direction, and when seen from a skin side the disposable diaper that is in a spread-out state with the absorbent main body (10) opened in the lengthwise direction at the predetermined location to unfold a twofold state of the absorbent main body,
the first elastic member (18) overlaps the absorbent core (11c) at the two end parts (11cea, 11ceb) of the absorbent core and simultaneously the first elastic member is located horizontally outward with respect to the absorbent core (11c) without overlapping the absorbent core at the constricted part (11ck).

6. An underpants-type disposable diaper according to any one of claims 1 to 5, further comprising
a barrier cuff (LSG) that can stand from a skin side face is supported by a support (HMA4) that is provided to the skin side face of the flap part (10f), wherein
a second elastic member (19) is provided along the lengthwise direction to a portion (10fp) that is between the support (HMA4) in the flap part (10f) and a horizontally outermost end (11cew) of the absorbent core (11c) to impart a lengthwise contraction force to the flap part (10f).

7. An underpants-type disposable diaper according to claim 6, wherein
the second elastic member (19) includes an effective portion (19v) that can impart the contraction force and ineffective portions (19iv) that are each located on two lengthwise sides of the effective portion (19v) and that cannot impart the contraction force and
the abdomen side waist part (31) includes a plurality of abdomen side elastic members (35) along the horizontal direction to impart a horizontal contraction force to the abdomen side waist part, and the abdomen side elastic member includes an effective portion (35v) that can impart the contraction force and ineffective portions (35iv) that are each located on two horizontal sides of the effective portion (35v) and cannot impart the contraction force,
when seen from a skin side the disposable diaper that is in a spread-out state with the absorbent main body (10) opened in the lengthwise direction at the predetermined location to unfold a twofold state of the absorbent main body,
an intersecting location (Pc19), where the effective portion (35v) of at least one abdomen side elastic member among the plurality of the abdomen side elastic members (35) intersects with the effective portion (19v) of the second elastic member (19), overlaps an area where the abdomen side waist part (31) is joined in the flap part (10f).

8. An underpants-type disposable diaper article according to any one of claims 1 to 7, wherein
a third elastic member (20) to impart the lengthwise contraction force to the flap part (10f) is provided in the lengthwise direction and extended across the Z-shape folded portion (10fZ) on the one side and the Z-shape folded portion (10fZ) on the other side,
the third elastic member includes an effective portion (20v) that can impart the contraction force and ineffective portions (20iv) that are each located on two lengthwise sides of the effective portion (20v) and that cannot impart the contraction force,
the abdomen side waist part (31) includes a plurality of abdomen side elastic members (35) along the horizontal direction to impart a horizontal contraction force to the abdomen side waist part, and the abdomen side elastic member includes an effective portion (35v) that can impart the contraction force and ineffective portions (35iv) that are each located on two horizontal sides of the effective portion and cannot impart the contraction force,
when seen from a skin side the disposable diaper that is in a spread-out state with the absorbent main body (10) opened in the lengthwise direction at the predetermined location to unfold a twofold state of the absorbent main body,
the effective portion (35v) of at least one abdomen side elastic member among the plurality of the abdomen side elastic members (35) does not intersect with the effective portion (20v) of the third elastic member (20) at an area where the abdomen side waist part (31) is joined in the flap part (10f).

9. An underpants-type disposable diaper according to any one of claims 1 to 8, wherein
the back side waist part (41) includes a plurality of back side elastic members (45) along the horizontal direction to impart a horizontal contraction force to the back side waist part, and
the back side elastic member (45) includes an effective portion (45v) that can impart the contraction force and ineffective portions (45iv) that are each located on two horizontal sides of the effective portion (45v) and cannot impart the contraction force,
when seen from a skin side the disposable diaper that is in a spread-out state with the absorbent main body (10) opened in the lengthwise direction at the predetermined location to unfold a twofold state of the absorbent main body,
at least a part of the effective portion (45v) of at least one back side elastic member among the plurality of the back side elastic members (45) is located at an area where the Z-shape folded portion of the flap part (10f) is joined in the back side waist part (41).

10. An underpants-type disposable diaper according to any one of claims 1 to 9, wherein
the Z-shape folded portion (10fZ) includes an inner fold part (10fZ1) that is folded back horizontally inward at a first folding location (P1) in the horizontal direction to extend horizontally inward with respect to the first folding location (P1) and an outer fold part (10fZ2) that is folded back horizontally outward at a second folding location (P2) in the inner fold part to extend horizontally outward with respect to the second folding location (P2),
the inner fold part (10fZ1) and the outer fold part (10fZ2) have faces opposing each other joined, and a horizontally inner portion (10fk) with respect to the first folding location (P1) in the flap part (10f) and the inner fold part (10fZ1) have faces opposing each other joined as well, in the Z-shape folded portion,
the flap part (10f) includes a liquid impermeable sheet (15a) and simultaneously the liquid impermeable sheet is arranged to extend in the horizontal direction toward the inner fold part (10fZ1) and the outer fold part (10fZ2) while a horizontal tip end part (10fZ2e) of the outer fold part is a non-positioned portion where the leak impermeable sheet is not arranged, and
the non-positioned portion has a fifth elastic member (20) provided along the lengthwise direction to impart a lengthwise contraction force to the non-positioned portion.

11. An underpants-type disposable diaper according to any one of claims 1 to 10, further comprising
the Z-shape folded portion (10fZ) includes an inner fold part (10fZ1) that is folded back horizontally inward at a first folding location (P1) in the horizontal direction to extend horizontally inward with respect to the first folding location (P1) and an outer fold part (10fZ2) that is folded back horizontally outward at a second folding location (P2) in the inner fold part to extend horizontally outward with respect to the second folding location (P2),
the flap part (10f) includes a middle portion (10fM) that is not fixed in a folded state at a location between the Z-shape folded portion (10fZ) on the one side and the Z-shape folded portion (10fZ) on the other side,
when a portion that is located between the inner fold part (10fZ1) on the one side and the inner fold part (10fZ1) on the other side in the middle portion is defined as a middle portion inner fold part (10fMZ1), and simultaneously a portion that is located between the outer fold part (10fZ2) on the one side and the outer fold part (10fZ2) on the other side in the middle portion is defined as a middle portion outer fold part (10fMZ2),
a single or a plurality of inner fold part elastic members (20a) that impart a lengthwise contraction force to the middle portion inner fold part (10fMZ11) are provided to extend along the lengthwise direction from the one side of the inner fold part (10fZ1) to the other side of the inner fold part (10fZ1),
a single or a plurality of outer fold part elastic members (20b)that impart a lengthwise contraction force to the middle portion outer fold part (10fMZ2) are provided to extend along the lengthwise direction from the one side of the outer fold part (10fZ2) to the other side of the outer fold part (10fZ2), and
a lengthwise contraction force (N/cm) per unit horizontal length imparted to the middle portion inner fold part (10fMZ1) by the inner fold part elastic members (20a) is greater than the lengthwise contraction force (N/cm) per the unit horizontal length imparted to the middle portion outer fold part (10fMZ2) by the outer fold part elastic members (20b).

## Patentansprüche

1. Höschenähnliche Einwegwindel (1), die eine vertikale Richtung und eine horizontale Richtung aufweist, die einander schneiden, die Folgendes umfasst:
einen absorbierenden Hauptkörper (10), der eine Längsrichtung aufweist und an einer vorgegebenen Stelle (CL10) in der Längsrichtung einmal gefaltet ist sowie die Längsrichtung derart aufweist, dass sie entlang der vertikalen Richtung angeordnet ist;
ein Bauchseitentaillenteil (31), das mit einer Nicht-Hautseitenfläche eines Endteils auf einer Seite in der Längsrichtung des absorbierenden Hauptkörpers überlappt und verbunden ist; und
ein Rückseitentaillenteil (41), das mit einer Nicht-Hautseitenfläche eines Endteils auf einer anderen Seite in der Längsrichtung des absorbierenden Hauptkörpers überlappt und verbunden ist; wobei
der absorbierende Hauptkörper (10) Folgendes einschließt: einen Saugkern (11c), der Flüssigkeit absorbiert, und ein lagenähnliches Klappenteil (10f), das sich horizontal nach außen in Bezug auf den Saugkern erstreckt,
das Klappenteil (10f) auf jeder von der einen Seite und der anderen Seite in der Längsrichtung einen in Z-Form gefalteten Abschnitt einschließt, der in einem Zustand befestigt ist, der entlang der horizontalen Richtung in einer Z-Form gefaltet vorliegt,
Abschnitte, die sich horizontal nach außen in Bezug auf den Saugkern (11c) in dem Bauchseitentaillenteil (31) und dem Rückseitentaillenteil (41) befinden, jeweils die in Z-Form gefalteten Abschnitte (10fZ) des Klappenteils befestigt aufweisen,
die Windel weiter ein Sperrbündchen (LSG) umfasst, das von einer Hautseitenfläche stehen kann und durch eine Stütze (HMA4) gestützt wird, die für die Hautseitenfläche des Klappenteils (10f) bereitgestellt ist,
das Klappenteil einen mittleren Abschnitt (10fM) einschließt, der nicht in einem gefalteten Zustand an einer Stelle zwischen dem in Z-Form gefalteten Abschnitt (lOfZ) auf der einen Seite und dem in Z-Form gefalteten Abschnitt (10fZ) auf der anderen Seite befestigt ist, und
eine maximale Länge (L10fM) von der Stütze (HMA4) zu einem horizontalen Spitzenendteil (10fMe) des mittleren Abschnitts, wenn der mittlere Abschnitt ungefaltet und in einem horizontal ausgebreiteten Zustand vorliegt, größer ist als eine maximale Länge (LLSG) von der Stütze (HMA4) zu einem horizontalen Spitzenendteil (LSGe) des Sperrbündchens.

2. Höschenähnliche Einwegwindel nach Anspruch 1, wobei
ein vorgegebener Abschnitt (10fZ2p1) ein horizontales äußeres Randteil in dem in Z-Form gefalteten Abschnitt (lOfZ) einschließt, wobei der vorgegebene Abschnitt, der einen innersten Abschnitt in der Längsrichtung eines Abschnitts einschließt, der mit dem Bauchseitentaillenteil (31) überlappt, ein nicht befestigter Abschnitt ist, der nicht an dem Bauchseitentaillenteil befestigt ist,
ein Abschnitt (10fZ2p3), nach innen benachbart zu dem nicht befestigten Abschnitt (10fZ2p1) in der horizontalen Richtung, in dem in Z-Form gefalteten Abschnitt einen befestigten Abschnitt einschließt, der zu dem Bauchseitentaillenteil zu befestigen ist, und
ein Abschnitt (10fZ2p2), nach außen benachbart zu dem nicht befestigten Abschnitt (10fZ2p1) in der Längsrichtung, in dem in Z-Form gefalteten Abschnitt einen befestigten Abschnitt einschließt, der zu dem Bauchseitentaillenteil (31) zu befestigen ist.

3. Höschenähnliche Einwegwindel nach Anspruch 1 oder 2, wobei
der in Z-Form gefaltete Abschnitt (lOfZ) Folgendes einschließt: ein inneres Faltteil (10fZ1), das an einer ersten Faltstelle (P1) in der horizontalen Richtung horizontal nach innen zurückgefaltet ist, um sich horizontal nach innen in Bezug auf die erste Faltstelle (P1) zu erstrecken, und ein äußeres Faltteil (10fZ2), das an einer zweiten Faltstelle (P2) in dem inneren Faltteil horizontal nach außen zurückgefaltet ist, um sich horizontal nach außen in Bezug auf die zweite Faltstelle (P2) zu erstrecken,
das innere Faltteil (10fZ1) und das äußere Faltteil (10fZ2) Flächen aufweisen, die einander gegenüberliegend verbunden sind, und ein horizontal innerer Abschnitt (10fk) in Bezug auf die erste Faltstelle (P1) in dem Klappenteil (10f) und das innere Faltteil (10fZ1) Flächen in dem in Z-Form gefalteten Abschnitt aufweisen, die auch einander gegenüberliegend verbunden sind, und
das äußere Faltteil (10fZ2) einen Abschnitt (10fZ2p) aufweist, der sich horizontal nach außen in Bezug auf das innere Faltteil (10fZ1) erstreckt.

4. Höschenähnliche Einwegwindel nach Anspruch 3, wobei
ein vorgegebener Abschnitt (10fZ2p1), der ein horizontales äußeres Randteil des sich erstreckenden Abschnitts (10fZ2p) in dem äußeren Faltteil einschließt und der weiter einen innersten Abschnitt in der Längsrichtung eines Abschnitts einschließt, der mit dem Bauchseitetaillenteil (31) überlappt, ein nicht befestigter Abschnitt ist, der nicht an dem Bauchseitentaillenteil befestigt ist,
ein Abschnitt (10fZ2p2), der nach außen benachbart zu dem nicht befestigten Abschnitt (10fZ2p1) in der Längsrichtung in dem sich erstreckenden Abschnitt (10fZ2p) in dem äußeren Faltteil (10fZ2) vorliegt, einen befestigten Abschnitt einschließt, der an dem Bauchseitentaillenteil (31) zu befestigen ist, und
ein Abschnitt (10fZ2p3), der nach innen benachbart zu dem nicht befestigten Abschnitt (10fZ2p1) in der horizontalen Richtung in dem in Z-Form gefalteten Abschnitt (lOfZ) vorliegt, einen befestigten Abschnitt einschließt, der an dem Bauchseitentaillenteil (31) zu befestigen ist.

5. Höschenähnliche Einwegwindel nach einem der Ansprüche 1 bis 4, wobei
der Saugkern (11c) ein verengtes Teil (11ck), das eine kleine horizontale Abmessung in Bezug auf zwei Endteile (11cea, 11ceb) in der Längsrichtung des Saugkerns aufweist, an einer Stelle zwischen den zwei Endteilen in der Längsrichtung einschließt und
der absorbierende Hauptkörper (10) ein erstes elastisches Element (18) entlang der Längsrichtung einschließt, um dem absorbierenden Hauptkörper eine Kontraktionskraft in der Längsrichtung zu verleihen, und bei Betrachtung von einer Hautseite der Einwegwindel, die in einem ausgebreiteten Zustand, mit dem absorbierenden Hauptkörper (10) in der Längsrichtung an der vorgegebenen Stelle geöffnet, vorliegt, um einen doppelten Zustand des absorbierenden Hauptkörpers zu entfalten,
das erste elastische Element (18) mit dem Saugkern (11c) an den zwei Endteilen (11cea, 11ceb) des Saugkerns überlappt und gleichzeitig sich das erste elastische Element horizontal nach außen in Bezug auf den Saugkern (11c) ohne Überlappung mit dem Saugkern an dem verengten Teil (11ck) befindet.

6. Höschenähnliche Einwegwindel nach einem der Ansprüche 1 bis 5, die weiter Folgendes umfasst:
ein Sperrbündchen (LSG), das von einer Hautseitenfläche stehen kann, wird durch eine Stütze (HMA4) gestützt, die für die Hautseitenfläche des Klappenteils (10f) bereitgestellt ist, wobei
ein zweites elastisches Element (19) entlang der Längsrichtung an einem Abschnitt (10fp) bereitgestellt ist, der zwischen der Stütze (HMA4) in dem Klappenteil (10f) und einem horizontal äußersten Ende (11cew) des Saugkerns (11c) vorliegt, um dem Klappenteil (10f) eine längsgerichtete Kontraktionskraft zu verleihen.

7. Höschenähnliche Einwegwindel nach Anspruch 6, wobei
das zweite elastische Element (19) Folgendes einschließt: einen wirksamen Abschnitt (19v), der die Kontraktionskraft verleihen kann, und unwirksame Abschnitte (19iv), die sich jeweils an zwei Längsseiten des wirksamen Abschnitts (19v) befinden und die keine Kontraktionskraft verleihen können, und
das Bauchseitentaillenteil (31) eine Vielzahl von elastischen Bauchseitenelementen (35) entlang der horizontalen Richtung einschließt, um dem Bauchseitentaillenteil eine horizontale Kontraktionskraft zu verleihen, und das elastische Bauchseitenelement Folgendes einschließt: einen wirksamen Abschnitt (35v), der die Kontraktionskraft verleihen kann, und unwirksame Abschnitte (35iv), die sich jeweils an zwei horizontalen Seiten des wirksamen Abschnitts (35v) befinden und keine Kontraktionskraft verleihen können,
bei Betrachtung von einer Hautseite der Einwegwindel, die in einem ausgebreiteten Zustand, mit dem absorbierenden Hauptkörper (10) in der Längsrichtung an der vorgegebenen Stelle geöffnet, vorliegt, um einen doppelten Zustand des absorbierenden Hauptkörpers zu entfalten,
eine Überschneidungsstelle (Pc19), wo der wirksame Abschnitt (35v) von mindestens einem elastischen Bauchseitenelement unter der Vielzahl von elastischen Bauchseitenelementen (35) den wirksamen Abschnitt (19v) des zweiten elastischen Elements (19) schneidet, mit einer Fläche überlappt, wo das Bauchseitentaillenteil (31) in dem Klappenteil (10f) verbunden ist.

8. Höschenähnlicher Einwegwindelartikel nach einem der Ansprüche 1 bis 7, wobei
ein drittes elastisches Element (20), um dem Klappenteil (10f) die längsgerichtete Kontraktionskraft zu verleihen, in der Längsrichtung bereitgestellt ist und sich über den in Z-Form gefalteten Abschnitt (lOfZ) auf der einen Seite und den in Z-Form gefalteten Abschnitt (lOfZ) auf der anderen Seite erstreckt,
das dritte elastische Element Folgendes einschließt: einen wirksamen Abschnitt (20v), der die Kontraktionskraft verleihen kann, und unwirksame Abschnitte (20iv), die sich jeweils an zwei Längsseiten des wirksamen Abschnitts (20v) befinden und die keine Kontraktionskraft verleihen können,
das Bauchseitentaillenteil (31) eine Vielzahl von elastischen Bauchseitenelementen (35) entlang der horizontalen Richtung einschließt, um dem Bauchseitentaillenteil eine horizontale Kontraktionskraft zu verleihen, und das elastische Bauchseitenelement Folgendes einschließt: einen wirksamen Abschnitt (35v), der die Kontraktionskraft verleihen kann, und unwirksame Abschnitte (35iv), die sich jeweils an zwei horizontalen Seiten des wirksamen Abschnitts befinden und keine Kontraktionskraft verleihen können,
bei Betrachtung von einer Hautseite der Einwegwindel, die in einem ausgebreiteten Zustand, mit dem absorbierenden Hauptkörper (10) in der Längsrichtung an der vorgegebenen Stelle geöffnet, vorliegt, um einen doppelten Zustand des absorbierenden Hauptkörpers zu entfalten,
der wirksame Abschnitt (35v) von mindestens einem elastischen Bauchseitenelement unter der Vielzahl der elastischen Bauchseitenelemente (35) nicht den wirksamen Abschnitt (20v) des dritten elastischen Elements (20) an einer Fläche schneidet, wo das Bauchseitentaillenteil (31) in dem Klappeteil (10f) verbunden ist.

9. Höschenähnliche Einwegwindel nach einem der Ansprüche 1 bis 8, wobei
das Rückseitentaillenteil (41) eine Vielzahl von elastischen Rückseitenelementen (45) entlang der horizontalen Richtung einschließt, um dem Rückseitentaillenteil eine horizontale Kontraktionskraft zu verleihen, und
das elastische Rückseitenelement (45) Folgendes einschließt: einen wirksamen Abschnitt (45v), der die Kontraktionskraft verleihen kann, und unwirksame Abschnitte (45iv), die sich jeweils an zwei horizontalen Seiten des wirksamen Abschnitts (45v) befinden und keine Kontraktionskraft verleihen können,
bei Betrachtung von einer Hautseite der Einwegwindel, die in einem ausgebreiteten Zustand, mit dem absorbierenden Hauptkörper (10) in der Längsrichtung an der vorgegebenen Stelle geöffnet, vorliegt, um einen doppelten Zustand des absorbierenden Hauptkörpers zu entfalten,
sich mindestens ein Teil des wirksamen Abschnitts (45v) von mindestens einem elastischen Rückseitenelement unter der Vielzahl der elastischen Rückseitenelemente (45) an einer Fläche befindet, wo der in Z-Form gefaltete Abschnitt des Klappenteils (10f) in dem Rückseitentaillenteil (41) verbunden ist.

10. Höschenähnliche Einwegwindel nach einem der Ansprüche 1 bis 9, wobei
der in Z-Form gefaltete Abschnitt (lOfZ) Folgendes einschließt: ein inneres Faltteil (10fZ1), das an einer ersten Faltstelle (P1) in der horizontalen Richtung horizontal nach innen zurückgefaltet ist, um sich horizontal nach innen in Bezug auf die erste Faltstelle (P1) zu erstrecken, und ein äußeres Faltteil (10fZ2), das an einer zweiten Faltstelle (P2) in dem inneren Faltteil horizontal nach außen zurückgefaltet ist, um sich horizontal nach außen in Bezug auf die zweite Faltstelle (P2) zu erstrecken,
das innere Faltteil (10fZ1) und das äußere Faltteil (10fZ2) Flächen aufweisen, die einander gegenüberliegend verbunden sind, und ein horizontal innerer Abschnitt (10fk) in Bezug auf die erste Faltstelle (P1) in dem Klappenteil (10f) und das innere Faltteil (10fZ1) Flächen in dem in Z-Form gefalteten Abschnitt aufweisen, die auch einander gegenüberliegend verbunden sind,
das Klappenteil (10f) eine flüssigkeitsundurchlässige Lage (15a) einschließt und gleichzeitig die flüssigkeitsundurchlässige Lage angeordnet ist, um sich in der horizontalen Richtung in Richtung des inneren Faltteils (10fZ1) und des äußeren Faltteils (10fZ2) zu erstrecken, während ein horizontales Spitzenendteil (10fZ2e) des äußeren Faltteils ein nicht positionierter Abschnitt ist, wo die auslaufundurchlässige Lage nicht angeordnet ist, und
der nicht positionierte Abschnitt ein fünftes elastisches Element (20) aufweist, das entlang der Längsrichtung bereitgestellt ist, um dem nicht positionierten Abschnitt eine längsgerichtete Kontraktionskraft zu verleihen.

11. Höschenähnliche Einwegwindel nach einem der Ansprüche 1 bis 10, die weiter Folgendes umfasst:
der in Z-Form gefaltete Abschnitt (lOfZ) schließt Folgendes ein: ein inneres Faltteil (10fZ1), das an einer ersten Faltstelle (P1) in der horizontalen Richtung horizontal nach innen zurückgefaltet ist, um sich horizontal nach innen in Bezug auf die erste Faltstelle (P1) zu erstrecken, und ein äußeres Faltteil (10fZ2), das an einer zweiten Faltstelle (P2) in dem inneren Faltteil horizontal nach außen zurückgefaltet ist, um sich horizontal nach außen in Bezug auf die zweite Faltstelle (P2) zu erstrecken,
das Klappenteil (10f) einen mittleren Abschnitt (10fM) einschließt, der nicht in einem gefalteten Zustand an einer Stelle zwischen dem in Z-Form gefalteten Abschnitt (lOfZ) auf der einen Seite und dem in Z-Form gefalteten Abschnitt (lOfZ) auf der anderen Seite befestigt ist,
wenn ein Abschnitt, der sich zwischen dem inneren Faltteil (10fZ1) auf der einen Seite und dem inneren Faltteil (10fZ1) auf der anderen Seite in dem mittleren Abschnitt befindet, als ein inneres Faltteil des mittleren Abschnitts (10fMZ1) definiert ist und gleichzeitig ein Abschnitt, der sich zwischen dem äußeren Faltteil (10fZ2) auf der einen Seite und dem äußeren Faltteil (10fZ2) auf der anderen Seite in dem mittleren Abschnitt befindet, als ein äußeres Faltteil des mittleren Abschnitts (10fMZ2) definiert ist,
ein einzelnes oder eine Vielzahl von elastischen Elementen des inneren Faltteils (20a), die dem inneren Faltteil des mittleren Abschnitts (10fMZ11) eine längsgerichtete Kontraktionskraft verleihen, bereitgestellt sind, um sich entlang der Längsrichtung von der einen Seite des inneren Faltteils (10fZ1) zu der anderen Seite des inneren Faltteils (10fZ1) zu erstrecken,
ein einzelnes oder eine Vielzahl von elastischen Elementen des äußeren Faltteils (20b), die dem äußeren Faltteil des mittleren Abschnitts (10fMZ2) eine längsgerichtete Kontraktionskraft verleihen, bereitgestellt sind, um sich entlang der Längsrichtung von der einen Seite des äußeren Faltteils (10fZ2) zu der anderen Seite des äußeren Faltteils (10fZ2) zu erstrecken, und
eine längsgerichtete Kontraktionskraft (N/cm) pro Einheit der horizontalen Länge, die dem inneren Faltteil des mittleren Abschnitts (10fMZ1) durch die elastischen Elemente des inneren Faltteils (20a) verliehen wird, größer ist als die längsgerichtete Kontraktionskraft (N/cm) pro Einheit der horizontalen Länge, die dem äußeren Faltteil des mittleren Abschnitts (10fMZ2) durch die elastischen Elemente des äußeren Faltteils (20b) verliehen wird.

## Revendications

1. Couche jetable de type culotte (1) ayant une direction verticale et une direction horizontale qui s'entrecoupent, comprenant :
une garniture absorbante principale (10) qui a une direction de longueur et qui est pliée en deux à un emplacement prédéterminé (CL10) dans la direction de longueur, et dont la direction de longueur est en outre disposée le long de la direction verticale ;
une partie de ceinture du côté abdominal (31) qui chevauche et qui est jointe à une surface qui n'est pas au contact de la peau d'une partie d'extrémité d'un côté dans la direction de longueur de la garniture absorbante principale ; et
une partie de ceinture du côté dorsal (41) qui chevauche et qui est jointe à une surface qui n'est pas au contact de la peau d'une partie d'extrémité d'un autre côté dans la direction de longueur de la garniture absorbante principale ; où
la garniture absorbante principale (10) inclut un noyau absorbant (11c) qui absorbe les liquides et une partie volet semblable à un feuillet (10f) qui se prolonge horizontalement vers l'extérieur par rapport au noyau absorbant,
la partie volet (10f) inclut, sur chacun du un côté et sur l'autre côté dans la direction de longueur, une portion pliée en forme de Z qui est fixe dans un état plié en forme de Z le long de la direction horizontale,
des portions situées horizontalement vers l'extérieur par rapport au noyau absorbant (11c) dans la partie de ceinture du côté abdominal (31) et la partie de ceinture du côté dorsal (41), respectivement, ont les portions pliées en forme de Z fixes (10fZ) de la partie volet,
la couche comprend en outre un revers faisant barrière (LSG) qui peut s'écarter d'une surface au contact de la peau et qui est soutenu par un support (HMA4) fourni au niveau de la surface au contact de la peau de la partie volet (10f),
la partie volet inclut une portion médiane (10fM) qui n'est pas fixe dans un état plié à un emplacement entre la portion pliée en forme de Z (10fZ) sur le un côté et la portion pliée en forme de Z (10fZ) sur l'autre côté et
une longueur maximale (L10fM) entre le support (HMA4) et une partie d'extrémité terminale horizontale (10fMe) de la portion médiane, quand la portion médiane est dépliée et dans un état horizontalement déployé, est plus importante qu'une longueur maximale (LLSG) entre le support (HMA4) et une partie d'extrémité terminale horizontale (LSGe) du revers faisant barrière.

2. Couche jetable de type culotte selon la revendication 1, où
une portion prédéterminée (10fZ2p1) inclut une partie formant bordure extérieure horizontale dans la portion pliée en forme de Z (10fZ), la portion prédéterminée, y compris une portion la plus interne dans la direction de longueur d'une portion qui chevauche la partie de ceinture du côté abdominal (31), étant une portion non fixe qui n'est pas fixée à la partie de ceinture du côté abdominal,
une portion (10fZ2p3) adjacente vers l'intérieur à la portion non fixe (10fZ2p1) dans la direction horizontale dans la portion pliée en forme de Z inclut une portion fixe qui est destinée à être fixée à la partie de ceinture du côté abdominal et
une portion (10fZ2p2) adjacente vers l'extérieur à la portion non fixe (10fZ2p1) dans la direction de longueur dans la portion pliée en forme de Z inclut une portion fixe qui est destinée à être fixée à la partie de ceinture du côté abdominal (31).

3. Couche jetable de type culotte selon la revendication 1 ou 2, où
la portion pliée en forme de Z (10fZ) inclut une partie de pliage interne (10fZ1) qui est rabattue horizontalement vers l'intérieur au niveau d'un premier emplacement de pliage (P1) dans la direction horizontale de manière à se prolonger horizontalement vers l'intérieur par rapport au premier emplacement de pliage (P1) et une partie de pliage externe (10fZ2) qui est rabattue horizontalement vers l'extérieur au niveau d'un deuxième emplacement de pliage (P2) dans la partie de pliage interne de manière à se prolonger horizontalement vers l'extérieur par rapport au deuxième emplacement de pliage (P2),
la partie de pliage interne (10fZ1) et la partie de pliage externe (10fZ2) ont des surfaces en opposition jointes l'une à l'autre, et une portion horizontalement interne (10fk) par rapport au premier emplacement de pliage (P1) dans la partie volet (10f) et la portion de pliage interne (10fZ1) ont des surfaces en opposition également jointes l'une à l'autre au niveau de la portion pliée en forme de Z et
la partie de pliage externe (10fZ2) a une portion (10fZ2p) qui se prolonge horizontalement vers l'extérieur par rapport à la partie de pliage interne (10fZ1).

4. Couche jetable de type culotte selon la revendication 3, où
une portion prédéterminée (10fZ2p1), qui inclut une partie formant bordure extérieure horizontale de la portion en prolongement (10fZ2p) dans la partie de pliage externe et qui inclut en outre une portion la plus interne dans la direction de longueur d'une portion qui chevauche la partie de ceinture du côté abdominal (31), est une portion non fixe qui n'est pas fixée à la partie de ceinture du côté abdominal,
une portion (10fZ2p2) qui est adjacente vers l'extérieur à la portion non fixe (10fZ2p1) dans la direction de longueur de la portion en prolongement (10fZ2p) dans la partie de pliage externe (10fZ2) inclut une portion fixe qui est destinée à être fixée à la partie de ceinture du côté abdominal (31) et
une portion (10fZ2p3) adjacente vers l'intérieur dans la direction horizontale à la portion non fixe (10fZ2p1) dans la portion pliée en forme de Z (10fZ) inclut une portion fixe qui est destinée à être fixée à la partie de ceinture du côté abdominal (31).

5. Couche jetable de type culotte selon l'une quelconque des revendications 1 à 4, où
le noyau absorbant (11c) inclut une partie resserrée (11ck) qui a une dimension horizontale faible par rapport aux deux parties d'extrémité (11cea, 11ceb) dans la direction de longueur du noyau absorbant à un emplacement entre les deux parties d'extrémité dans la direction de longueur et
la garniture absorbante principale (10) inclut un premier organe élastique (18) le long de la direction de longueur pour transmettre une force de contraction à la garniture absorbante principale dans la direction de longueur et, quand la couche jetable qui est dans un état déployé avec la garniture absorbante principale (10) ouverte dans la direction de longueur à l'emplacement prédéterminé est visualisée depuis un côté au contact de la peau, pour déplier un état doublement replié de la garniture absorbante principale,
le premier organe élastique (18) chevauche le noyau absorbant (11c) aux deux parties d'extrémité (11cea, 11ceb) du noyau absorbant et simultanément le premier organe élastique est situé horizontalement vers l'extérieur par rapport au noyau absorbant (11c) sans chevaucher le noyau absorbant au niveau de la partie resserrée (11ck).

6. Couche jetable de type culotte selon l'une quelconque des revendications 1 à 5 comprenant en outre un revers faisant barrière (LSG) qui peut s'écarter d'une surface au contact de la peau et qui est soutenu par un support (HMA4) fourni au niveau de la surface au contact de la peau de la partie volet (10f), où
un deuxième organe élastique (19) est fourni le long de la direction de longueur (10fp) au niveau d'une portion située entre le support (HMA4) dans la partie volet (10f) et une extrémité horizontalement le plus à l'extérieur (11cew) du noyau absorbant (11c) pour transmettre une force de contraction longitudinale à la partie volet (10f).

7. Couche jetable de type culotte selon la revendication 6, où
le deuxième organe élastique (19) inclut une portion efficace (19v) qui peut transmettre la force de contraction et des portions inefficaces (19iv) qui sont chacune situées sur deux côtés longitudinaux de la portion efficace (19v) et qui ne peuvent pas transmettre la force de contraction et
la partie de ceinture du côté abdominal (31) inclut une pluralité d'organes élastiques du côté abdominal (35) le long de la direction horizontale pour transmettre une force de contraction horizontale à la partie de ceinture du côté abdominal, et l'organe élastique du côté abdominal inclut une portion efficace (35v) qui peut transmettre la force de contraction et des portions inefficaces (35iv) qui sont chacune situées sur deux côtés horizontaux de la portion efficace (35v) et qui ne peuvent pas transmettre la force de contraction,
quand la couche jetable qui est dans un état déployé avec la garniture absorbante principale (10) ouverte dans la direction de longueur à l'emplacement prédéterminé est visualisée depuis un côté au contact de la peau, pour déplier un état doublement replié de la garniture absorbante principale,
un emplacement d'intersection (Pc19), au niveau duquel la portion efficace (35v) de un au moins organe élastique du côté abdominal parmi la pluralité d'organes élastiques du côté abdominal (35) entrecroise la portion efficace (19v) du deuxième organe élastique (19), chevauche une région au niveau de laquelle la partie de ceinture du côté abdominal (31) est jointe à la partie volet (10f).

8. Couche jetable de type culotte selon l'une quelconque des revendications 1 à 7, où
un troisième organe élastique (20) pour transmettre la force de contraction longitudinale à la partie volet (10f) est fourni dans la direction de longueur et se prolonge au travers de la portion pliée en forme de Z (10fZ) sur un côté et de la portion pliée en forme de Z (10fZ) sur l'autre côté,
le troisième organe élastique inclut une portion efficace (20v) qui peut transmettre la force de contraction et des portions inefficaces (20iv) qui sont chacune situées sur les deux côtés longitudinaux de la portion efficace (20v) et qui ne peuvent pas transmettre la force de contraction,
la partie de ceinture du côté abdominal (31) inclut une pluralité d'organes élastiques du côté abdominal (35) le long de la direction horizontale pour transmettre une force de contraction horizontale à la partie de ceinture du côté abdominal, et l'organe élastique du côté abdominal inclut une portion efficace (35v) qui peut transmettre la force de contraction et des portions inefficaces (35iv) qui sont chacune situées sur deux côtés horizontaux de la portion efficace et qui ne peuvent pas transmettre la force de contraction,
quand la couche jetable qui est dans un état déployé avec la garniture absorbante principale (10) ouverte dans la direction de longueur à l'emplacement prédéterminé est visualisée depuis un côté au contact de la peau, pour déplier un état doublement replié de la garniture absorbante principale,
la portion efficace (35v) de un au moins organe élastique du côté abdominal parmi la pluralité d'organes élastiques du côté abdominal (35) n'entrecroise pas la portion efficace (20v) du troisième organe élastique (20) dans une région au niveau de laquelle la partie de ceinture du côté abdominal (31) est jointe à la partie volet (10f).

9. Couche jetable de type culotte selon l'une quelconque des revendications 1 à 8, où
la partie de ceinture du côté dorsal (41) inclut une pluralité d'organes élastiques du côté dorsal (45) le long de la direction horizontale pour transmettre une force de contraction horizontale à la partie de ceinture du côté dorsal et
l'organe élastique du côté dorsal (45) inclut une portion efficace (45v) qui peut transmettre la force de contraction et des portions inefficaces (45iv) qui sont chacune situées sur deux côtés horizontaux de la portion efficace (45v) et qui ne peuvent pas transmettre la force de contraction,
quand la couche jetable qui est dans un état déployé avec la garniture absorbante principale (10) ouverte dans la direction de longueur à l'emplacement prédéterminé est visualisée depuis un côté au contact de la peau, pour déplier un état doublement replié de la garniture absorbante principale,
au moins une partie de la portion efficace (45v) de un au moins organe élastique du côté dorsal parmi la pluralité d'organes élastiques du côté dorsal (45) est située dans une région au niveau de laquelle la portion pliée en forme de Z de la partie volet (10f) est jointe à la partie de ceinture du côté dorsal (41).

10. Couche jetable de type culotte selon l'une quelconque des revendications 1 à 9, où
la portion pliée en forme de Z (10fZ) inclut une partie de pliage interne (10fZ1) qui est rabattue horizontalement vers l'intérieur au niveau d'un premier emplacement de pliage (P1) dans la direction horizontale de manière à se prolonger horizontalement vers l'intérieur par rapport au premier emplacement de pliage (P1) et une partie de pliage externe (10fZ2) qui est rabattue horizontalement vers l'extérieur au niveau d'un deuxième emplacement de pliage (P2) dans la partie de pliage interne de manière à se prolonger horizontalement vers l'extérieur par rapport au deuxième emplacement de pliage (P2),
la partie de pliage interne (10fZ1) et la partie de pliage externe (10fZ2) ont des surfaces en opposition jointes l'une à l'autre, et une portion horizontalement interne (10fk) par rapport au premier emplacement de pliage (P1) dans la partie volet (10f) et la portion de pliage interne (10fZ1) ont des surfaces en opposition également jointes l'une à l'autre au niveau de la portion pliée en forme de Z,
la partie volet (10f) inclut un feuillet imperméable aux liquides (15a) et simultanément le feuillet imperméable aux liquides est disposé de manière à se prolonger dans la direction horizontale vers la partie de pliage interne (10fZ1) et la partie de pliage externe (10fZ2) tandis qu'une partie d'extrémité terminale horizontale (10fZ2e) de la partie de pliage externe est une portion non positionnée au niveau de laquelle le feuillet imperméable aux liquides n'est pas disposé et
la portion non positionnée a un cinquième organe élastique (20) fourni le long de la direction de longueur pour transmettre une force de contraction longitudinale à la portion non positionnée.

11. Couche jetable de type culotte selon l'une quelconque des revendications 1 à 10, comprenant en outre
la portion pliée en forme de Z (10fZ) inclut une partie de pliage interne (10fZ1) qui est rabattue horizontalement vers l'intérieur au niveau d'un premier emplacement de pliage (P1) dans la direction horizontale de manière à se prolonger horizontalement vers l'intérieur par rapport au premier emplacement de pliage (P1) et une partie de pliage externe (10fZ2) qui est rabattue horizontalement vers l'extérieur au niveau d'un deuxième emplacement de pliage (P2) dans la partie de pliage interne de manière à se prolonger horizontalement vers l'extérieur par rapport au deuxième emplacement de pliage (P2),
la partie volet (10f) inclut une portion médiane (10fM) qui n'est pas fixe dans un état plié à un emplacement entre la section pliée en forme de Z (10fZ) sur le un côté et la portion pliée en forme de Z (10fZ) sur l'autre côté,
où une portion qui est située entre la partie de pliage interne (10fZ1) sur le un côté et la partie de pliage interne (10fZ1) sur l'autre côté dans la portion médiane est définie en tant que partie de pliage interne de la portion médiane (10fMZ1) et simultanément une portion qui est située entre la partie de pliage externe (10fZ2) sur le un côté et la partie de pliage externe (10fZ2) sur l'autre côté dans la portion médiane est définie en tant que partie de pliage interne de la portion médiane (10fMZ2),
un unique ou une pluralité d'organes élastiques de la partie de pliage interne (20a) qui transmettent une force de contraction longitudinale à la partie de pliage interne de la portion médiane (10fMZ11) sont fournis de manière à se prolonger le long de la direction de longueur depuis le un côté de la partie de pliage interne (10fZ1) jusqu'à l'autre côté de la partie de pliage interne (10fZ1),
un unique ou une pluralité d'organes élastiques de la partie de pliage externe (20b) qui transmettent une force de contraction longitudinale à la partie de pliage externe de la portion médiane (10fMZ2) sont fournis de manière à se prolonger le long de la direction de longueur depuis le un côté de la partie de pliage externe (10fZ2) jusqu'à l'autre côté de la partie de pliage externe (10fZ2) et
une force de contraction longitudinale (N/cm) par unité de longueur horizontale transmise à la partie de pliage interne de la portion médiane (10fMZ1) par les organes élastiques de la partie de pliage interne (20a) est plus élevée que la force de contraction longitudinale (N/cm) par unité de longueur horizontale transmise à la partie de pliage externe de la portion médiane (10fMZ2) par les organes élastiques de la partie de pliage externe (20b).
